# EUROPEAN PATENT SPECIFICATION

(11) **EP 3 815 658 B1**
(45) Date of publication and mention of the grant of the patent: **04.03.2026**
(21) Application number: 19825989.7
(22) Date of filing: 27.06.2019
(51) Int. Cl.: A61F 13/49, A61F 13/51

(54) **ABSORBENT ITEM AND SHEET MEMBER**
ABSORBIERENDER ARTIKEL UND BLATTELEMENT
ARTICLE ABSORBANT ET ÉLÉMENT DE FEUILLE

(30) Priority: 29.06.2018 JP 2018125606
(43) Date of publication of application: 05.05.2021
(73) Proprietor: Unicharm Corporation, Shikokuchuo-shi, Ehime 799-0111 (JP)
(72) Inventor: YOSHIOKA, Toshiyasu, Kanonji-shi, Kagawa 769-1602 (JP); FUKASAWA, Jun, Kanonji-shi, Kagawa 769-1602 (JP)
(74) Representative: Staeger & Sperling Partnerschaftsgesellschaft mbB
(86) International application number: PCT/JP2019/025718
(87) International publication number: WO 2020/004589

(56) References cited:
- EP-A1- 3 357 464
- WO-A1-2016/093370
- WO-A1-2017/056937
- WO-A1-2018/030482
- JP-A- 2016 067 501
- JP-A- 2018 089 519
- US-A1- 2006 228 969
- US-A1- 2012 259 307

## Description

### [Technical Field]

The present invention relates to an absorbent article and a sheet member.

### [Background Art]

Patent Literature 1 discloses an absorbent article having not only a thread-like or band-like elastic member but also a stretching/contracting sheet having stretchability. The stretching/contracting sheet has a surface that faces the wearer's skin and is thus capable of compressing the wearer's skin with the surface. A region in which the stretching/contracting sheet is disposed can be brought into close contact with the wearer's body with the surface. As compared with the thread-like or band-like elastic member, the stretching/contracting sheet is less likely to cause a local tightening and can suppress deterioration of wearer's feeling of wearing.

### [Patent Literature]

Patent Literature 1:Japanese Unexamined Patent Application Publication No. 2015-202226. Further prior art in this technical field is disclosed in documents US 2006/228969 A1, US 2012/259307 A1 and EP 3 357 464 A1.

### [Summary of the Invention]

### [Technical Problem]

It is desired to change compression by a place (for example, a center of a belly portion and a side portion of the belly portion) in a predetermined direction (for example, a width direction) so as not to deteriorate the wearer's feeling of wearing while bringing an absorbent article into close contact with a body in order to hold the absorbent article to the body. In a region in which the stretching/contracting sheet is disposed, it is possible to increase compression in a region in which the absorbent article is brought into close contact with the body and to decrease compression in a region in which it is necessary to cover the body with the absorbent article along the shape, thereby changing the compression in a predetermined direction.

Here, in order to change the compression by the stretching/contracting sheet, the compression can be changed in a predetermined direction by arranging the region in which the stretching/contracting sheet is disposed and the region in which the stretching/contracting sheet is not disposed. However, since the compression is excessively low in the region in which the stretching/contracting sheet is not disposed, the absorbent article cannot be firmly brought into close contact with the body.

**It** is also considered to change the compression by arranging a plurality of types of stretching/contracting sheets having different stretching forces in a predetermined direction. However, when a plurality of types of stretching/contracting sheets are used, the number of types of materials increases, so that a manufacturing process becomes complicated. In addition, at the time of manufacturing an absorbent article, particularly at the time of manufacturing an absorbent article by an apparatus which has been increased in speed, since the stretching/contracting sheet is difficult to control as compared with a thread-like or band-like elastic member, the production difficulty is enhanced by using the plurality of types of stretching/contracting sheets. Therefore, there is a problem that the production cost as a whole increases.

Accordingly, there has been desired an absorbent article including a stretching/contracting sheet and a thread-like or band-like elastic member, in which the absorbent article is capable of being held by a body and suppressing deterioration of the wearer's feeling of wearing by changing the compression in a predetermined direction while suppressing an increase in the production cost, and a sheet member used for the absorbent article.

### [Solution to Problem]

The present invention is defined in claim 1 and refers to an absorbent article.

A sheet member according to the present invention is defined in claim 10.

### [Brief Description of Drawings]

Fig. 1 is a plan view of an absorbent article according to an embodiment when viewed from a skin surface side.
Fig. 2 is a plan view of the absorbent article according to the embodiment when viewed from the skin surface side.
Fig. 3 is a cross-sectional view taken along an A-A line illustrated in Fig. 1.
Fig. 4 is a cross-sectional view taken along a line B-B illustrated in Fig. 1.
Fig. 5 is a cross-sectional view taken along a line C-C illustrated in Fig. 1.
Fig. 6 is a view for describing the absorbent article according to the embodiment.
Fig. 7 is a view schematically illustrating a putting state of the absorbent article according to the embodiment.
Fig. 8 is a view schematically illustrating the putting state of the absorbent article according to the embodiment.
Fig. 9 is a view schematically illustrating a conveyance state of the absorbent article according to the embodiment during the manufacturing of the absorbent article.

### [Description of Embodiments]

### (1) Summary of embodiment

At least following matters will become clear with description of this specification and attached drawings.

According to an aspect, there is provided an absorbent article having a first direction, a second direction orthogonal to the first direction, a thickness direction orthogonal to the first direction and the second direction, includes a stretching/contracting sheet having stretchability, and a thread-like or band-like elastic member disposed to overlap with the stretching/contracting sheet in the thickness direction in a stretched state in the first direction. The absorbent article includes a sheet region having contractility in the first direction by at least the stretching/contracting sheet. The sheet region includes a high contraction sheet region in which the elastic member is disposed so as to overlap with the stretching/contracting sheet in the thickness direction, and a low contraction sheet region having lower contractility than that of the high contraction sheet region in the first direction. The high contraction sheet region and the low contraction sheet region are arranged in the first direction.

According to this aspect, in the high contraction sheet region, the elastic member is disposed to overlap the stretching/contracting sheet, and thereby it is possible to increase the contractive force. On the other hand, in the low contraction sheet region, the contractive force is lower than that of the high contraction sheet region in the first direction. The low contraction sheet region and the high contraction sheet region are arranged in the first direction, and thereby it is possible to change compression in the first direction. Therefore, it is possible to increase compression in a region where it is necessary to closely attach the absorbent article to the body and to decrease compression in a region where it is necessary to cover the body with the absorbent article along the shape. As a result, it is possible to hold the absorbent article on the body and to suppress the deterioration of the wearer's feeling of wearing. In addition, since the thread-like or band-like elastic member is easier to control during the manufacturing of the absorbent article than the stretching/contracting sheet, it is possible to change the compression in the sheet region in the first direction without enhancing the production difficulty. Therefore, it is possible to hold the absorbent article on the body while suppressing an increase in production costs and to suppress the deterioration of the wearer's feeling of wearing.

According to a preferable aspect, the low contraction sheet region is disposed between the high contraction sheet regions in the first direction. Since the low contraction sheet region is disposed between the high contraction sheet regions, the high contraction sheet region can be brought into close contact with the body by the contraction of the high contraction sheet regions on both sides of the low contraction sheet region in the first direction. Therefore, it is possible to make it difficult for the low contraction sheet region that is disposed between the high contraction sheet regions to separate from the body. Therefore, in the low contraction sheet region, it is possible to hold the absorbent article on the body along the shape of the body.

According to a preferable aspect, the sheet region includes a first region in which the high contraction sheet region and the low contraction sheet region are arranged in the first direction, and a second region in which only the high contraction sheet region is disposed in the first direction. The second region and at least the low contraction sheet region of the first region are arranged in the second direction. Here, at the time of manufacturing the absorbent article, the sheet member is conveyed in a state of being stretched in the first direction so that the sheet member (for example, a continuous sheet continuous in the first direction) does not have wrinkles in the first direction in some cases. Due to stretching at this time, the sheet member, the width of the sheet member may be reduced in the orthogonal direction perpendicular to the first direction (so-called neck-in), it may not be stably conveyed the sheet member. Therefore, the low contraction sheet region in the first region and the second region in which only the high contraction sheet region is disposed are arranged in the second direction, whereby a region of the low contraction sheet region alone is no longer present in the second direction. That is, the high contraction sheet region is continuously disposed in the first direction. Since the force that stretches the sheet member acts in a direction in which the high contraction sheet region HSR is stretched, the contractive force of the high contraction sheet region acts against the force. Therefore, the force for stretching the sheet member is weakened, and the neck-in can be reduced.

According to the invention, the first direction is a width direction, and the second direction is a front-back direction. The absorbent article has a first waistline region, a second waistline region, a crotch region sandwiched between the first waistline region and the second waistline region in the front-back direction, and an exterior body disposed in the first waistline region and having the sheet region. In the first waistline region, the high contraction sheet region and the low contraction sheet region can be arranged in the width direction. Therefore, it is possible to provide a region in which the exterior body is easily closely attached to the wearer's waistline and a region in which the exterior body is easily attached to the wearer's waistline along the shape, and it is possible to continuously hold the exterior body on the body while suppressing the deterioration of the wearer's feeling of wearing. The exterior body does not slip down, and it is possible to continuously hold the absorbent article on the body.

According to the invention, the first waistline region is a front waistline region. The low contraction sheet region is disposed to straddle the center of the exterior body in the width direction. The high contraction sheet regions are respectively located at outer side of the low contraction sheet region in the width direction. The low contraction sheet region is disposed to straddle the center of the exterior body in the width direction and is thus disposed at the center of the wearer's belly portion in the width direction. Regarding the shape of the wearer's belly portion at the center in the width direction, generally, the belly swells from the recess at the lower end of the belly portion toward the upper side (outer side in the front-back direction). The low contraction sheet region is disposed against the swelling of the belly, whereby it is possible to suppress the excessive close attachment of the exterior body to the swelling of the belly and to suppress the deterioration of the wearer's feeling of wearing. The contraction of the high contraction sheet regions makes it possible to closely attach the absorbent article to the body from the respective outer sides of the low contraction sheet region in the width direction with the high contraction sheet regions. Therefore, it is possible to make it difficult for the low contraction sheet region to separate from the body.

According to a preferable aspect, both outer side edges of the low contraction sheet region in the width direction are located at outer side in the width direction as the low contraction sheet region extends outer side in the front-back direction. Regarding the shape of the wearer's belly portion at the center in the width direction, the belly swells from the recess at the lower end of the belly portion toward the upper side (outer side in the front-back direction). Therefore, since both outer side edges of the low contraction sheet region are located at outer side in the width direction as the low contraction sheet region extends outer side in the front-back direction, it is possible to dispose the low contraction sheet region corresponding to the swelling of the belly. It is possible to suppress the excessive close attachment of the exterior body to the swelling of the belly and to suppress the deterioration of the wearer's feeling of wearing.

According to a preferable aspect, a length in the width direction of an outer end edge of the low contraction sheet region in the front-back direction is twice or more a length in the width direction of the inner end edge of the low contraction sheet region in the front-back direction. Regarding the shape of the wearer's belly portion at the center in the width direction, the belly swells from the recess at the lower end of the belly portion toward the upper side (outer side in the front-back direction). Therefore, the length in the width direction of the waistline region on the upper side of the belly portion is longer than the length in the width direction of the waistline region on the lower side of the belly portion. Accordingly, the length in the width direction of the outer side edge of the low contraction sheet region is set to twice or more the length in the width direction of the inner end edge of the low contraction sheet region, whereby it is possible to dispose the low contraction sheet region to correspond to the swelling of the belly. It is possible to suppress the excessive close attachment of the exterior body to the swelling of the belly and to suppress the deterioration of the wearer's feeling of wearing.

According to one preferable aspect, there is an absorbent main body which has an absorbent core and is disposed at least in the crotch region. At least a part of the outer side edge of the low contraction sheet region is located at outer side of an outer side edge of the absorbent main body in the width direction. Regarding the shape of the wearer's belly portion at the center in the width direction, the belly swells from the recess at the lower end of the belly portion toward the upper side (outer side in the front-back direction). Therefore, there are many cases where the swelling of the belly reaches outer side in the width direction beyond the outer side edges of the absorbent main body. Therefore, at least a part of the outer side edge of the low contraction sheet region is located at outer side of the outer side edges of the absorbent main body in the width direction, whereby it is possible to dispose the low contraction sheet region in a region including the swelling of the belly that is located at outer side of the absorbent main body in the width direction. It is possible to suppress the excessive close attachment of the exterior body to the swelling of the belly and to suppress the deterioration of the wearer's feeling of wearing.

According to a preferable aspect, the absorbent article includes an absorbent main body which has an absorbent core and is disposed at least in the crotch region. A part of the high contraction sheet regions overlaps with the absorbent main body in the thickness direction. It is possible to dispose the low contraction sheet region against the swelling of the belly and to closely attach the absorbent main body to the body by the contraction of the high contraction sheet regions. Therefore, the leakage of the excrement can be suppressed.

According to a preferable aspect, a part of the high contraction sheet region overlaps with the absorbent core in the thickness direction. It is possible to dispose the low contraction sheet region against the swelling of the belly and to closely attach the absorbent core to the body by the contraction of the high contraction sheet regions. Therefore, the leakage of the excrement can be suppressed.

According to a preferable aspect, one or more cuts are formed in the stretching/contracting sheet that configures the low contraction sheet region. Since the contractive force does not act in the stretching/contracting sheet portion in which one or more cuts are formed, it is possible to further reduce the contractive force of the stretching/contracting sheet in the low contraction sheet region. It is possible to attach the absorbent article to the body along the shape more flexibly and to further suppress the deterioration of the wearer's feeling of wearing.

According to an aspect, there is provided a sheet member according to claim 10.

### (2) Overall schematic configuration of absorbent article

Hereinafter, an absorbent article according to an embodiment will be described with reference to drawings. It should be noted that, in the following description of the drawings, identical or similar portions will be given identical or similar reference numerals. However, it should be noted that the drawings are schematic drawings and the ratios and the like of individual dimensions are different from the actual ones. Accordingly, specific dimensions and the like are supposed to be determined in consideration of the following description. In addition, the relationships or ratios of the dimensions may vary among the drawings.

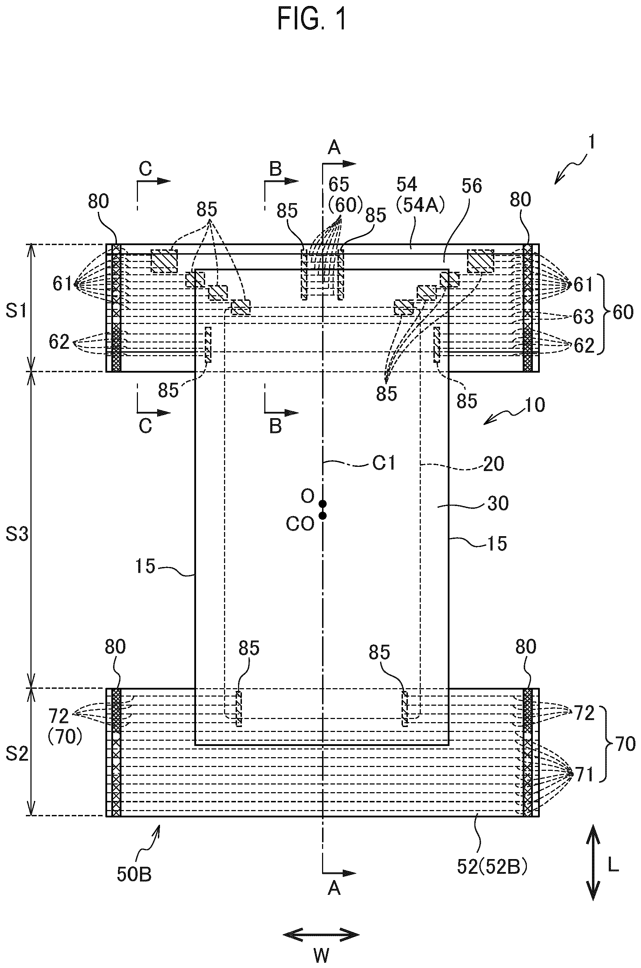

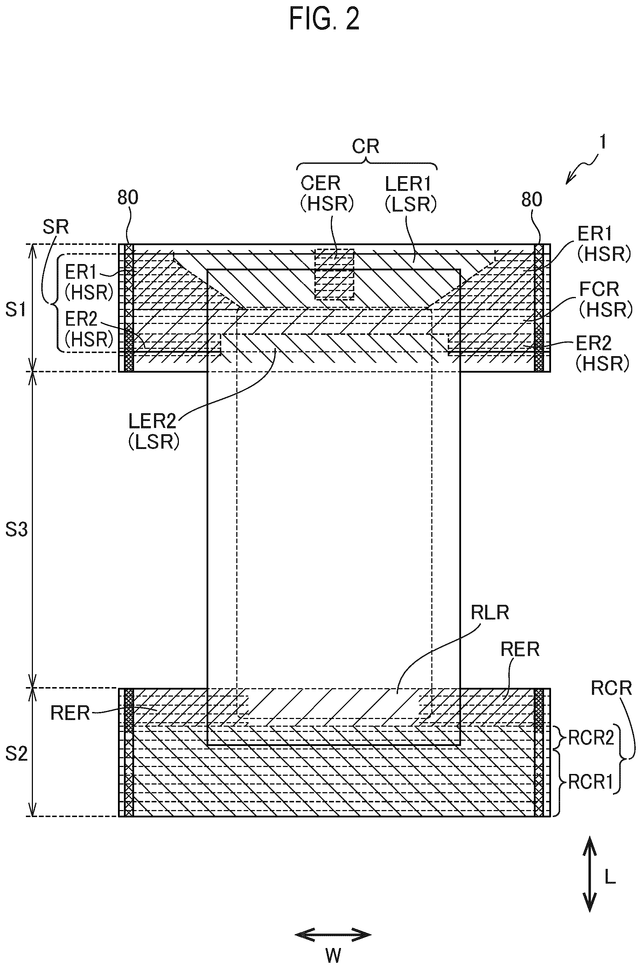

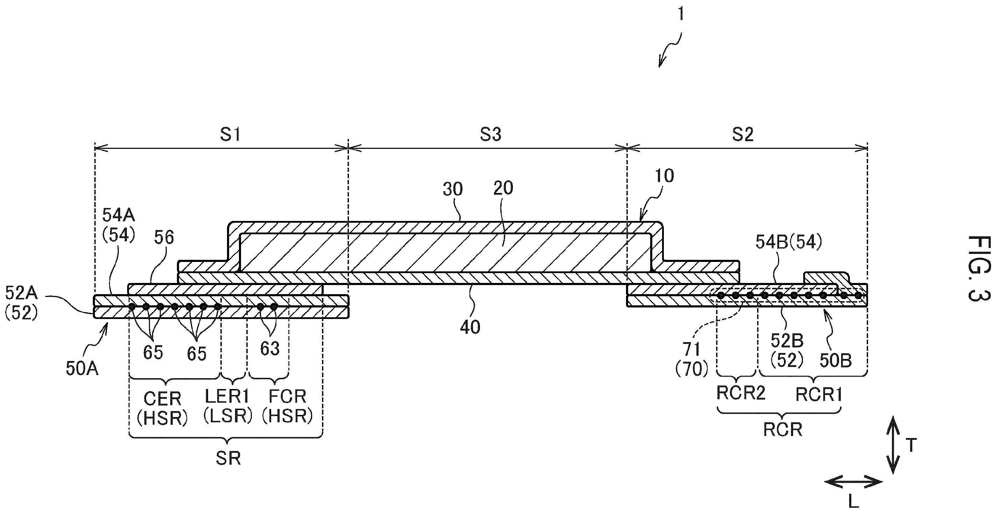

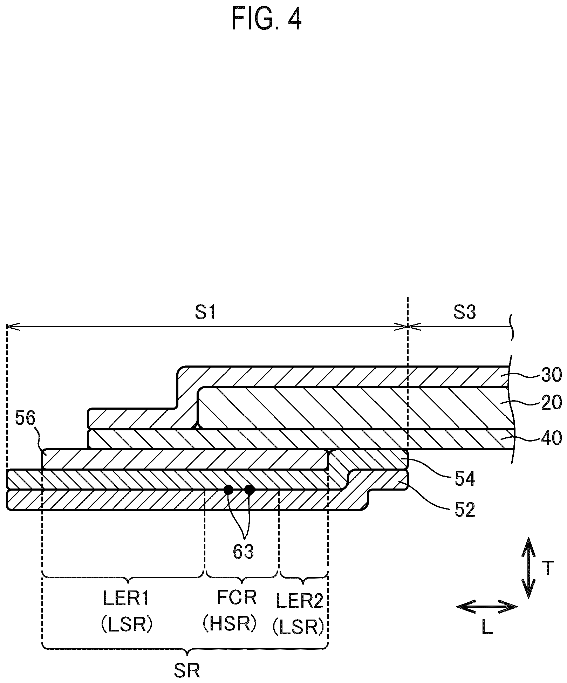

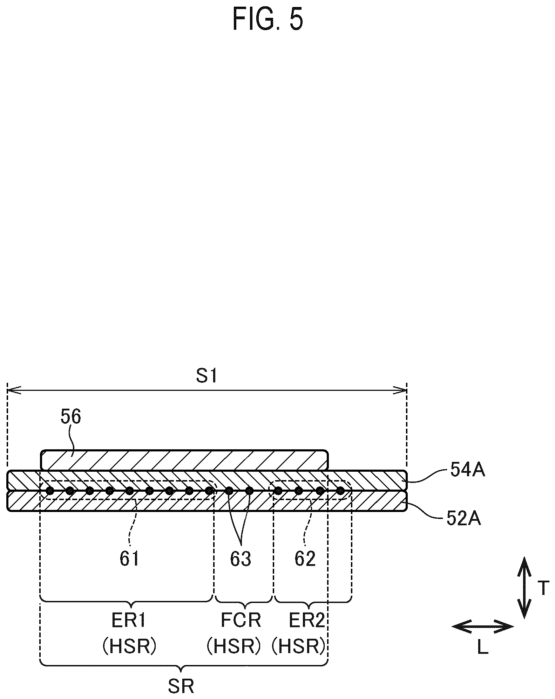

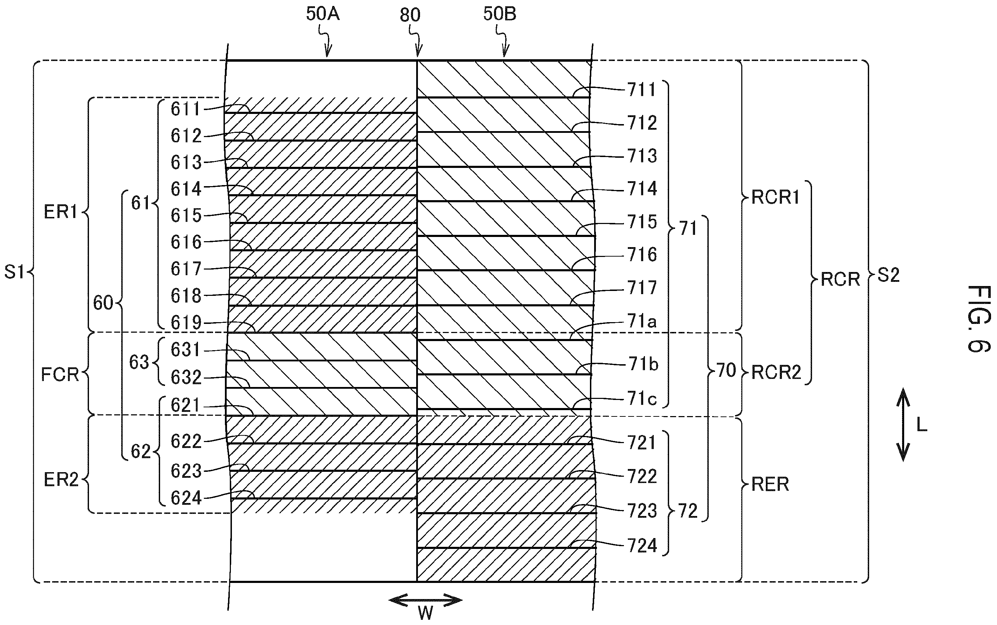

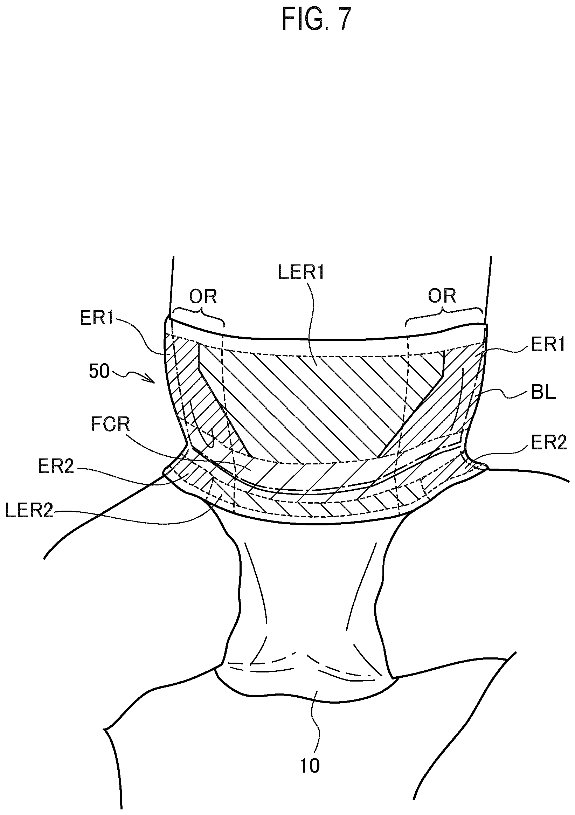

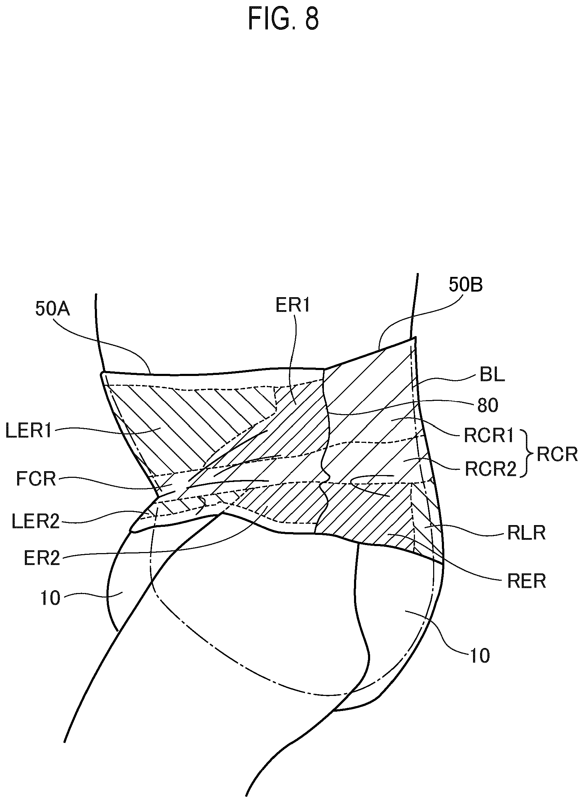

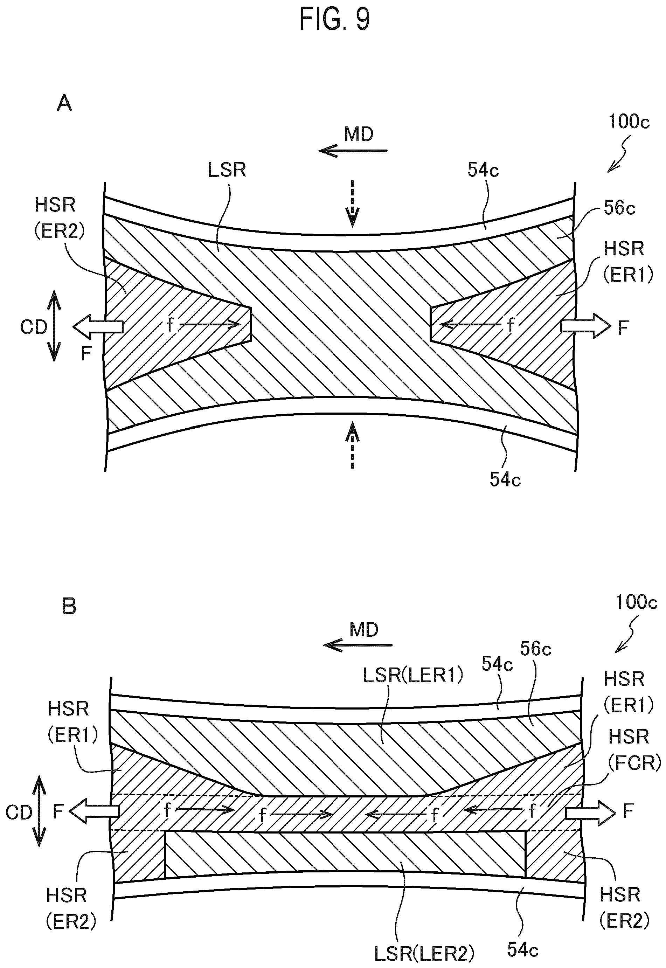

The overall schematic configuration of an absorbent article 1 will be described using Fig. 1 to Fig. 6. Fig. 1 is a plan view of the absorbent article according to the embodiment when viewed from the skin surface side. In detail, Fig. 1 and Fig. 2 are schematic plan views of the absorbent article 1 according to the present embodiment. The schematic plan views illustrated in Fig. 1 and Fig. 2 illustrate a stretched state of the absorbent article 1 in a stretched state in which the absorbent article 1 is stretched until wrinkles attributed to the contraction or the like of an elastic member or the like are not formed in an unfolded state in which side joining portions 80, which will be described below, are not folded. Fig. 2 schematically illustrates the region in a front waistline region S1 and the region in a rear waistline region S2 of the absorbent article 1. Fig. 3 is a cross-sectional view taken along a line A-A illustrated in Fig. 1. Fig. 4 is a cross-sectional view taken along a line B-B illustrated in Fig. 1. Fig. 5 is a cross-sectional view taken along a line C-C illustrated in Fig. 1. Fig. 6 is a view for describing the absorbent article according to the embodiment. In detail, Fig. 6 is a view illustrating of parts the front waistline region S1 and the rear waistline region S2 of the absorbent article 1 in a state in which the side joining portions 80 described below are folded. Fig. 7 and Fig. 8 are views schematically illustrating a putting state of the absorbent article according to the embodiment. The single-dotted chain line BL in Fig. 7 and Fig. 8 indicates the wearer's body line. In Fig. 7 and Fig. 8, it should be noted that a central contraction region CER, which will be described below, is not shown. Fig. 9 is a view schematically illustrating a conveyance state of the absorbent article according to the embodiment during the manufacturing of the absorbent article.

The absorbent article may be a tape type or a pants type. The absorbent article may be a disposable diaper or an underpants-type sanitary napkin. The absorbent article of the embodiment is a pants-type disposable diaper.

As illustrated in Fig. 1, the absorbent article 1 has a front-back direction L and a width direction W that are orthogonal to each other. The front-back direction L is defined by a direction extending toward the front side of the body and the back side of the body. In other words, the front-back direction L is a direction extending toward the front and the back in the unfolded absorbent article 1. In addition, the absorbent article 1 has a thickness direction T that is orthogonal to both the front-back direction L and the width direction W.

The absorbent article 1 has the front waistline region S1, the rear waistline region S2, and a crotch region S3. The front waistline region S1 is a region that faces the front waistline (belly portion) of the wearer. The rear waistline region S2 is a region that faces the back waistline (back portion) of the wearer. The crotch region S3 is a region that is located under the wearer's crotch and is disposed between the front waistline region S1 and the rear waistline region S2. Therefore, the crotch region S3 is sandwiched between the front waistline region S1 and the rear waistline region S2 in the front-back direction.

The crotch region S3 may be defined by a region in which leg openings 15 are provided. The leg opening 15 is a portion that is recessed inner side in the width direction W from the outer side edge of the disposable diaper. The boundary between the front waistline region S1 and the crotch region S3 may be defined by the front end edge of the leg opening 15. The boundary between the rear waistline region S2 and the crotch region S3 may be defined by the rear end edge of the leg opening 15.

In the present embodiment, the absorbent article 1 may have an absorbent main body 10 and an exterior body 50.

The absorbent main body 10 is disposed at least in the crotch region S3. The absorbent main body 10 may extend across the front waistline region S1, the rear waistline region S2, and the crotch region S3. Therefore, the absorbent main body 10 may be disposed to straddle a front exterior body 50A and a rear exterior body 50B. The absorbent main body 10 may be configured as a separate component from the front exterior body 50A and the rear exterior body 50B. In this case, the absorbent main body 10 may be joined to the front exterior body 50A and the rear exterior body 50B respectively in the front waistline region S1 and the rear waistline region S2.

The absorbent main body 10 has an absorbent core 20. The absorbent core 20 may be formed of an absorbent material that absorbs liquid. The absorbent core 20 may include, for example, pulp (pulverized pulp) or superabsorbent polymer (SAP) or a mixture thereof. The absorbent core 20 may have a region having a lower basis weight than the periphery (low-basis-weight portion) in a region that overlaps a front continuous contraction region FCR, which will be described below.

The basis weight can be measured according to an ordinary method. For example, (1) a range to be measured is marked, the area: SAα (m2) of the range is measured, (2) the marked range is cut out with a sharp knife, for example, a cutter, the total mass: TM (g) of the cut-out marked range is measured, and (3) the basis weight BSα (g/m2) of the range to be measured can be calculated based on the following equation: BSα (g/m2) = TM (g)/ SAα (m2).

The absorbent core 20 is disposed at least in the crotch region S3. Preferably, the absorbent core 20 may extend from the front waistline region S1 across the rear waistline region S2 in the front-back direction L. The absorbent core 20 may be covered with a core wrap (not shown). The core wrap can be formed of, for example, a nonwoven fabric or a tissue sheet.

The absorbent main body 10 may have a top-surface sheet 30 located on the skin surface side of the absorbent core 20 and a back-surface sheet 40 located on the non-skin surface side of the absorbent core 20.

The top-surface sheet 30 is a liquid-permeable sheet. The top-surface sheet 30 is formed of any sheet-like material having a structure that allows liquid to pass through, such as a nonwoven fabric, a woven fabric, a perforated plastic sheet, or a mesh sheet. As the material of the woven fabric or the nonwoven fabric, any of natural fiber and chemical fiber can be used.

The back-surface sheet 40 is a liquid-impermeable sheet. As the back-surface sheet 40, it is possible to use a polyethylene sheet, a laminated nonwoven fabric mainly containing polypropylene or the like, a breathable resin film, a sheet having a breathable resin film joined to a spunbond or spunlace nonwoven fabric, or the like.

The exterior body 50 is a member that is disposed in the waistline region. The exterior body 50 may also be referred to as a waistline member. The exterior body 50 has the front exterior body 50A and the rear exterior body 50B. The front exterior body 50A is disposed in the front waistline region S1. The rear exterior body 50B is disposed in the rear waistline region S2.

As illustrated in Fig. 1 and Fig. 3, the front exterior body 50A according to the present embodiment may have an exterior sheet 52 (front exterior sheet 52A), a side top sheet 54 (front side top sheet 54A), a stretching/contracting sheet 56, and a front elastic member 60.

The front exterior sheet 52A may be disposed on the non-skin surface side compared with the absorbent main body 10. The front exterior sheet 52A can be formed of, for example, a nonwoven fabric. The outer end edge of the front exterior sheet 52A in the front-back direction L may be at the same location as the outer end edge of the front side top sheet 54A in the front-back direction L. Similar to a rear exterior sheet 52B, which will be described below, the outer side end portion of the front exterior sheet 52A in the front-back direction L may be folded toward the non-skin surface side.

The front side top sheet 54A may be disposed on the skin surface side compared with the front exterior sheet 52A. The front side top sheet 54A can be formed of, for example, a nonwoven fabric. The front exterior sheet 52A and the front side top sheet 54A may be joined to each other with, for example, a sonic seal in a region where the front elastic member 60 is not disposed (low contraction region LER, which will be described below).

The stretching/contracting sheet 56 may be disposed on the skin surface side compared with the front side top sheet 54A. The stretching/contracting sheet 56 is a sheet-like elastic member. The stretching/contracting sheet 56 has stretchability at least in the width direction W. Therefore, the stretching/contracting sheet has contractility in the width direction W. The stretching/contracting sheet 56 may have stretchability (contractility) in the front-back direction L. The stretching/contracting sheet 56 is disposed to overlap the front elastic member 60 in the thickness direction T.

In a stretched state in which the stretching/contracting sheet 56 is stretched in the width direction W, the end portions of the stretching/contracting sheet 56 in the width direction W may be fixed to the front side top sheet 54A with the side joining portions 80. In the stretched state in which the stretching/contracting sheet 56 is stretched in the width direction W, at least a part of the main surface of the stretching/contracting sheet 56 may be fixed to the front side top sheet 54A with, for example, an adhesive portion (not shown) such as a hot-melt adhesive (HMA) or a sonic seal.

The stretching/contracting sheet 56 may be formed of, for example, a resin film having stretchability, a nonwoven fabric having stretchability, or a combination thereof.

The front elastic member 60 is a thread-like or band-like elastic member. The front elastic member 60 has contractility. In detail, since the front elastic member 60 has stretchability, the front elastic member 60 has contractility by being disposed in a stretched state. The front elastic member 60 is disposed in the stretched state of being stretched in the width direction W in the front waistline region S1. The front elastic member 60 may be disposed between the front exterior sheet 52A and the front side top sheet 54A in the thickness direction T. A plurality of the front elastic members 60 may be disposed side by side at intervals in the front-back direction L. As illustrated in Fig. 3 to Fig. 5, in the present embodiment, the front elastic member 60 is disposed to overlap the stretching/contracting sheet 56 in the thickness direction T in the stretched state of being stretched in the width direction W.

The front elastic member 60 may have first front elastic members 61, second front elastic members 62, a third front elastic member 63, and a central elastic member 65. The first front elastic members 61 are elastic members that are disposed at outer side of a central region CR (first low contraction region LER1), which will be described below, in the width direction W and are disposed at outer side of the third front elastic member 63 in the front-back direction L. The second front elastic members 62 are elastic members that are disposed at outer side of a second low contraction region LER2, which will be described below, in the width direction W and are disposed at inner side of the third front elastic member 63 in the front-back direction L. The third front elastic member 63 is an elastic member that continuously extends in the width direction W from one side portion toward the other side portion of the front waistline region S1 (front exterior body 50A) in the width direction W. The central elastic member 65 is an elastic member that is disposed in the central region CR, which will be described below.

The first front elastic members 61 may extend inner side in the width direction W from both side portions of the front waistline region S1 (front exterior body 50A) in the width direction W. The first front elastic members 61 do not continuously extend in the width direction W from one side portion toward the other side portion of the front waistline region S1 in the width direction W. The first front elastic members 61 may be fixed (adhere) to the side portions of the front waistline region S1 in the width direction W with the side joining portions 80. The inner end portion of the first front elastic member 61 in the width direction W may be fixed (adhere) to the front exterior sheet 52A with, for example, an adhesive portion 85 such as a hot-melt adhesive (HMA). The length of the first front elastic member 61 in the width direction W may decrease toward the outer side in the front-back direction L.

As illustrated in Fig. 6, the first front elastic member 61 of the present embodiment includes first front elastic members 611 to 619. In the present embodiment, the lengths of the first front elastic member 611 and the first front elastic member 612 in the width direction W are the same. In the present embodiment, the lengths of the first front elastic members 612 to 619 in the width direction W decrease toward the outer side in the front-back direction L. Therefore, the length in the width direction W of any first front elastic member 61 that is included in the first front elastic member 61 is equal to or longer than the length of the first front elastic member 61 located at the outer side of any first front elastic member 61 in the front-back direction L.

The second front elastic members 62 may extend inner side in the width direction W from both side portions of the front waistline region S1 (front exterior body 50A) in the width direction W. The second front elastic members 62 do not continuously extend in the width direction W from one side portion through the other side portion of the front waistline region S1 in the width direction W. The second front elastic members 62 may be fixed (adhere) to the side portions of the front waistline region S1 in the width direction W with the side joining portions 80. The inner end portion of the second front elastic member 62 in the width direction W may be fixed (adhere) to the front exterior sheet 52A with the adhesive portion 85. The lengths of the respective second front elastic members 62 in the width direction W may be the same as or different from each other. As illustrated in Fig. 6, the second front elastic member 62 of the present embodiment includes second front elastic members 621 to 624.

The third front elastic member 63 is disposed between the first front elastic member 61 and the second front elastic member 62 in the front-back direction L. The third front elastic member 63 may be fixed (adhere) to both side portions of the front waistline region S1 in the width direction W with the side joining portions 80. As illustrated in Fig. 6, the third front elastic member 63 of the present embodiment includes third front elastic members 631 and 632.

The central elastic member 65 is disposed between the first front elastic member 61 on one side and the first front elastic member 61 on the other side in the width direction W. The central elastic member 65 is disposed apart from the first front elastic member 61 in the width direction W. In the case of being provided by cutting the first front elastic member 61 as described below, the central elastic member 65 may be formed of the same member as the first front elastic member 61. The central elastic member 65 may be formed of a member different from the first front elastic member 61.

As illustrated in Fig. 1 and Fig. 3, the rear exterior body 50B according to the present embodiment may have an exterior sheet 52 (rear exterior sheet 52B), a side top sheet 54 (rear side top sheet 54B), and a rear elastic member 70.

The rear exterior sheet 52B may be disposed on the non-skin surface side compared with the absorbent main body 10. The rear exterior sheet 52B can be formed of, for example, a nonwoven fabric. The outer side end portion of the rear exterior sheet 52B in the front-back direction L may be folded toward the non-skin surface side. Therefore, at the end portion of the rear exterior body 50B in the front-back direction L, the rear exterior sheet 52B may sandwich the rear side top sheet 54B in the thickness direction T.

The rear side top sheet 54B may be disposed on the skin surface side compared with the rear exterior sheet 52B. The rear side top sheet 54B can be formed of, for example, a nonwoven fabric.

The rear elastic member 70 is a thread-like or band-like elastic member. The rear elastic member 70, similar to the front elastic member 60, has contractility. The rear elastic member 70 is disposed in the stretched state of being stretched in the width direction W in the rear waistline region S2. The rear elastic member 70 may be disposed between the rear exterior sheet 52B and the rear side top sheet 54B in the thickness direction T. The rear elastic members 70 may be disposed side by side in the front-back direction L.

The rear elastic member 70 may have a first rear elastic member 71 and second rear elastic members 72. The first rear elastic member 71 is an elastic member that is disposed in a rear continuous contraction region RCR, which will be described below. The second rear elastic members 72 are elastic members that are disposed in rear contraction regions RER, which will be described below.

The first rear elastic member 71 continuously extends in the width direction W from one side portion through the other side portion of the rear waistline region S2 in the width direction W. The first rear elastic member 71 may be fixed (adhere) to both side portions of the rear waistline region S2 in the width direction W with the side joining portions 80. As illustrated in Fig. 6, the first rear elastic member 71 of the present embodiment includes first rear elastic members 711 to 717 and first rear elastic members 71a to 71c. The first rear elastic members 711 to 717 are elastic members that are disposed in a first rear continuous contraction region RCR1, which will be described below. The first rear elastic members 71a to 71c are elastic members that are disposed in a second rear continuous contraction region RCR2, which will be described below. Therefore, the first rear elastic members 711 to 717 are not adjacent to the front continuous contraction region FCR in the width direction W, but the first rear elastic members 71a to 71c are adjacent to the front continuous contraction region FCR in the width direction W.

The second rear elastic members 72 may extend inner side in the width direction W from both side portions of the rear waistline region S2 (rear exterior body 50B) in the width direction W. The second rear elastic members 72 do not continuously extend in the width direction W from one side portion through the other side portion of the rear waistline region S2 in the width direction W. The second rear elastic members 72 are disposed on the inner side of the first rear elastic member 71 in the front-back direction L. The second rear elastic members 72 may be fixed (adhere) to the side portions of the rear waistline region S2 in the width direction W with the side joining portions 80. The inner end portion of the second rear elastic member 72 in the width direction W may be fixed (adhere) to the rear exterior sheet 52B with an adhesive portion 85. The lengths of the respective second rear elastic members 72 in the width direction W may be identical to or different from each other. As illustrated in Fig. 6, the second rear elastic member 72 of the present embodiment includes second rear elastic members 721 to 724.

The side joining portions 80 join the outer side portions of the front exterior body 50A (front waistline region S1) in the width direction W and the outer side portions of the rear exterior body 50B (rear waistline region S2) in the width direction W. The side joining portions 80 may extend along the front-back direction L. In the front-back direction L, the outer end edge of the side joining portion 80 in the front-back direction L may be at the same location as or at a different location from the outer end edge of the front exterior body 50A in the front-back direction L. In a case where the side joining portion 80 is provided, the outer end edge of the side joining portion 80 in the front-back direction L may be defined as the outer end edge of the front exterior body 50A in the front-back direction L. In the front-back direction L, the inner end edge of the side joining portion 80 in the front-back direction L may be at the same location as or at a different location from the inner end edge of the front exterior body 50A in the front-back direction L. In a case where the side joining portion 80 is provided, the inner end edge of the side joining portion 80 in the front-back direction L may be defined as the inner end edge of the front exterior body 50A in the front-back direction L.

Next, the region in the waistline region of the absorbent article 1 will be described using Fig. 2 to Fig. 6. First, the region in the front waistline region S1 (front exterior body 50A) will be described. In the present embodiment, the front waistline region S1 (front exterior body 50A) has outer regions OR that are located at outer side in the width direction W distanced from the center in the width direction W as a reference in a stretched state of the front exterior body 50A for more than 1/4 of the length in the width direction W of the front waistline region S1 (front exterior body 50A). In addition, the front waistline region S1 (front exterior body 50A) has a contraction region having contractility and a low contraction region having a contractive force lower than the contractive force of the contraction region. The low contraction region may be disposed to straddle the center of the front exterior body 50A in the width direction W. In the present embodiment, the low contraction region extends at outer side in the width direction W from the center in the width direction W. In addition, the contraction region has contractility due to the front elastic member 60. The contraction region is located at outer side of the low contraction region in the width direction W. Hereinafter, the low contraction region and the contraction region will be described in detail.

As illustrated in Fig. 2 and the like, the front waistline region S1 (front exterior body 50A) has first front contraction regions ER1, a central region CR (a first low contraction region LER1 and a central contraction region CER), a front continuous contraction region FCR, second front contraction regions ER2, and a second low contraction region LER2. The contraction region has the front continuous contraction region FCR, the first front contraction regions ER1, and the second front contraction regions ER2, and the low contraction region has the first low contraction region LER1 and the second low contraction region LER2.

In the present embodiment, the first front contraction regions ER1 are two regions that have contractility due to the front elastic member 60 (first front elastic member 61) and extend inner side in the width direction W from both side portions of the front waistline region S1 in the width direction W. Therefore, the first front contraction regions ER1 are disposed at outer side of the central region CR in the width direction W. That is, the first front contraction regions ER1 are disposed at outer side of the first low contraction region LER1 in the width direction W. In addition, in the present embodiment, the first front contraction regions ER1 are located at outer side of the front continuous contraction region FCR in the front-back direction L. Therefore, the first front contraction regions ER1 are disposed at outer side of the second front contraction regions ER2 in the front-back direction L.

The first front contraction regions ER1 are regions that contract due to the first front elastic members 611 to 619. Therefore, in the first front contraction regions ER1, the first front elastic members 611 to 619 are disposed. In addition, in the present embodiment, the first front contraction regions ER1 may have contractility due to the stretching/contracting sheet 56.

The inner edge of the first front contraction region ER1 in the width direction may be located further at outer side in the width direction W as the first front contraction region ER1 extends outer side in the front-back direction L. At least a part of the inner edge of the first front contraction region ER1 may be located at inner side of the outer side edge of the absorbent main body 10 in the width direction W. In the embodiment, a region that configures at outer side of the first front contraction region ER1 in the front-back direction L is located at outer side of the outer side edge of the absorbent main body 10 in the width direction W. On the other hand, a region that configures the inner side of the first front contraction region ER1 in the front-back direction L is located at inner side of the outer side edge of the absorbent main body 10 in the width direction W. The length in the width direction of the outer end edge of the first front contraction region ER1 in the front-back direction L may be twice or more the length in the width direction W of the inner end edge of the first front contraction region ER1 in the front-back direction L. (A part of) the first front contraction region ER1 may be disposed to overlap the absorbent main body 10 in the thickness direction T. (A part of) the first front contraction region ER1 may be disposed to overlap the absorbent core 20 in the thickness direction T. Since it is possible to closely attach the absorbent core 20 to a wearer's body by the contraction of the first front contraction regions ER1, the leakage of excrement can be suppressed. In addition, the maximum width in the width direction W of a region where the first front contraction region ER1 and the absorbent core 20 overlap may be 10 mm or less. In such a case, it is possible to suppress the shrinkage of the absorbent core 20 in the width direction W and to secure the absorption area of the absorbent core 20. In other embodiments, the first front contraction regions ER1 may be disposed so as not to overlap the absorbent core 20 in the thickness direction T.

The central region CR is a region that is disposed between the two first front contraction regions ER1 in the width direction W. At least a part of the central region CR may be disposed at outer side of the absorbent main body 10 in the front-back direction L. As illustrated in Fig. 2 and the like, in the present embodiment, the central region CR has a first low contraction region LER1 and a central contraction region CER.

The first low contraction region LER1 is a region in which the contractive force is reduced compared with the contractive force of the first front contraction region ER1. In the first low contraction region LER1, strong fastening by the front elastic member 60 is reduced. Therefore, (the first low contraction region LER1) in the front exterior body 50A that is disposed in the front waistline region S1 is capable of covering the wearer's body along the shape of the body, which can suppress the deterioration of the wearer's feeling of wearing.

It should be noted that, in the first low contraction region LER1, the contractive force can be reduced using, for example, a first method or a second method.

In the first method, the contraction region may be reduced in the first low contraction region LER1 by not disposing the front elastic member 60 in a stretched state. For example, in the first low contraction region LER1, the adhesive portion 85 for fixing the front elastic member 60 is not disposed, and the front elastic member 60 is fixed with the adhesive portion 85 and the side joining portion 80 in the stretched state of being stretched in the width direction W. In a case where the stretched front elastic member 60 overlaps (a region corresponding to) the first low contraction region LER1 in the thickness direction T, the front elastic member 60 is cut on the first low contraction region LER1. As a result, the cut front elastic member 60 contracts, and in the first low contraction region LER1, the front elastic member 60 is not arranged in the stretched state. In such a case, since the front elastic member 60 having contractility (that is, in a stretched state) is not disposed in the first low contraction region LER1, it becomes easy to control the contractive force in the first low contraction region LER1. It should be noted that, in a case where the first low contraction region LER1 is provided by cutting the front elastic member 60 as described above, the front elastic member 60 may have a fixed portion and a non-fixed portion.

The fixed portion is a portion that is fixed in a stretched state in the front waistline region S1. The fixed portion may be a portion that is fixed with the adhesive portion 85 and the side joining portion 80. The fixed portion may be the first front elastic member 61 that is disposed in the first front contraction region ER1 and the central elastic member 65 that is disposed in the central contraction region CER.

The non-fixed portion is a portion that is present in a natural state without being fixed in the front waistline region S1. The non-fixed portion is a portion that is disposed between the adhesive portions 85 before the cutting of the front elastic member 60, is a portion in which the front elastic member 60 contracts by cutting, and is continuous with the fixed portion of the front elastic member 60. The non-fixed portion may be disposed in the front exterior body 50A in a contracted state (natural state) through the adhesive portion 85. The non-fixed portion may overlap the first low contraction region LER1 in the thickness direction T. The non-fixed portion of the front elastic member 60 may be conveyed or the like during manufacturing to overlap the first front contraction region ER1 in the thickness direction T.

It should be noted that, in a case where the contractive force is reduced by the first method, the front elastic member 60 is not fixed in a stretched state to the first low contraction region LER1. Therefore, the first low contraction region LER1 does not overlap the thread-like or band-like front elastic member 60 in a stretched state in the thickness direction T.

In the second method, the contractive force of the front elastic member 60 may be reduced by cutting (cutting into lumps) the front elastic member 60 fixed to the first low contraction region LER1 at a plurality of places. In this case, even when the front elastic member 60 is fixed to the first low contraction region LER1 in a stretched state, the contractive force of the front elastic member 60 is reduced.

Each front elastic member 60 may be cut by the first method or the second method, which has been described above, after each front elastic member 60 is extended from one first front contraction region ER1 through the first low contraction region LER1 to the other first front contraction region ER1. In such a case, it is possible to dispose the same front elastic member 60 in two first front contraction regions ER1. Therefore, it is possible to dispose the front elastic members 60 having the same elongation ratio in one first front contraction region ER1 and the other first front contraction region ER1.

In addition, similarly, each front elastic member 60 may be cut by the first method or the second method, which has been described above, after each front elastic member 60 is extended from one first front contraction region ER1 through the first low contraction region LER1 and the central contraction region CER to the other first front contraction region ER1. In such a case, it is possible to dispose the same front elastic member 60 in two first front contraction regions ER1 and the central contraction region CER. Since it is not necessary to dispose separate front elastic members 60 in the plurality of contraction regions (the two first front contraction regions ER1 and the central contraction region CER) respectively, a reduction of the production efficiency can be suppressed.

The range of the first low contraction region LER1 (and the first front contraction regions ER1) may be defined by providing the first low contraction region by the above-described method. The "contractive force" of each region may be measured by the following method. When the contractive force of each region is measured, a test piece, which is a measurement object, is made. Both end portions of the test piece are clamped with chucks (clamping devices) of a tensile tester. At this time, the distance between the chucks is set to 50 mm. Next, in a state in which one of the chucks in the width direction W is fixed, the other chuck is moved in a manner such that the distance between the chucks changes. The movement speed of the chuck at this time is set to 300 mm/min. During the movement of the chuck, stress that is applied to the chuck is measured, and a stress (N) obtained in a state in which the width of the test piece reaches 60% (constant width) of the width in the stretched state is defined as the "contractive force".

The first low contraction region LER1 is located at outer side of the front continuous contraction region FCR in the front-back direction L. Therefore, the first low contraction region LER1 is disposed at outer side of the second low contraction region LER2 in the front-back direction L.

The first low contraction region LER1 may reach the outer end portion of the front exterior body 50A (front waistline region S1) in the front-back direction L. Therefore, the first low contraction region LER1 may be disposed to extend up to the outer end portion of the front exterior body 50A (front waistline region S1) in the front-back direction L. As illustrated in Fig. 7 and Fig. 8, since the belly swells from the recess at the lower end of the belly portion toward the upper side (outer side in the front-back direction), the waistline length of the belly becomes longer at the outer end portion of the front exterior body 50A in the front-back direction L, and, as the waistline region becomes longer, the belly is likely to be fastened more strongly. Since the first low contraction region LER1 is disposed to extend up to the outer end portion of the front exterior body 50A in the front-back direction L, it is possible to reduce fastening in a portion where the belly is likely to be fastened more strongly with the first low contraction region LER1, which can suppress the deterioration of the wearer's feeling of wearing.

The outer end portion of the front exterior body 50A in the front-back direction L is a region up to a maximum of 10 mm from the outer end edge of the front exterior body 50A in the front-back direction L. At the outer end portion of the front exterior body 50A in the front-back direction L, one front elastic member 60 extending in the width direction W may or may not be disposed. The front elastic member 60 may extend from one side portion up to the other side portion of the front exterior body 50A in the width direction W.

The first low contraction region LER1 may be disposed between the central contraction region CER and the first front contraction region ER1 in the width direction W. The maximum length in the width direction W of the first low contraction region LER1 may be longer than the length in the width direction W of the central contraction region CER. In such a case, it is possible to secure the first low contraction region LER1, which is a region in which the contractive force is reduced compared with the contractive force of the central contraction region CER, and to suppress the contraction of the absorbent cores 20 in the width direction W more than necessary due to the contractive force of the central contraction region CER. Therefore, it is possible to secure the absorption area of the absorbent core 20 while securing rigidity in the central region CR.

In the present embodiment, the outer side edges of the first low contraction region LER1 in the width direction W are disposed in the outer regions OR outer side of the absorbent main body 10 in the front-back direction L. On the outer side of the absorbent main body 10 in the front-back direction L, a part of the outer side edges of the first low contraction region LER1 may be disposed in the outer regions OR, or all of the outer side edges of the first low contraction region LER1 may be disposed in the outer regions OR. As illustrated in Fig. 7, the swelling of the belly gradually broadens towards outer side in the width direction W as the belly swells from the recess at the lower end of the wearer's belly portion toward the upper side (outer side in the front-back direction). When the outer side edges of the first low contraction region LER1 that extends to outer side in the width direction W from the center in the width direction W are disposed in the outer regions OR, it is easy to dispose the low contraction region in a location corresponding to the shape of the swelling of the belly. Therefore, it is possible to reduce strong fastening not only in the center of the belly portion in the width direction but also in the side portions of the belly portion on the upper side of the belly portion, which can suppress the deterioration of the wearer's feeling of wearing. In addition, since the first front contraction regions ER1 are located at outer side of the first low contraction region LER1 in the width direction, the front exterior body 50A is closely attached to the wearer's body by the contraction of the first front contraction regions ER1, whereby the slip-down of the front exterior body 50A toward the crotch side can be suppressed.

In the present embodiment, the length from the center of the front exterior body 50A in the width direction W to the outer side edge of the first low contraction region LER1 in the width direction W is maximized on the outermost side of the first low contraction region LER1 in the front-back direction L. Therefore, as illustrated in Fig. 7, it is possible to reduce strong fastening not only in the center of the belly portion in the width direction but also in the side portions of the belly portion on the upper side of the belly portion, which can suppress the deterioration of the wearer's feeling of wearing.

One outer side edge of the first low contraction region LER1 in the width direction W may be located further at outer side in the width direction W as the first low contraction region LER1 extends outer side in the front-back direction L. Therefore, the outer side edge of the first low contraction region LER1 in the width direction W may be located further at inner side in the width direction W as the first low contraction region LER1 extends inner side in the front-back direction L. In addition, both outer side edges of the first low contraction region LER1 in the width direction W may be located further at outer side in the width direction W as the first low contraction region LER1 extends outer side in the front-back direction L. Therefore, both outer side edges of the first low contraction region LER1 in the width direction W may be located further at inner side in the width direction W as the first low contraction region LER1 extends towards inner side in the front-back direction L. As illustrated in Fig. 7, the swelling of the belly gradually broadens outer side in the width direction W as the belly swells from the recess at the lower end of the belly portion toward the upper side (outer side in the front-back direction). Both outer side edges of the first low contraction region LER1 are located further at outer side in the width direction W as the first low contraction region LER1 extends outer side in the front-back direction, whereby it is possible to dispose the first low contraction region LER1 in the region corresponding to the shape of the swelling of the belly. The first low contraction region LER1 corresponds to the shape of the belly, whereby it is possible to suppress excessive close attachment to the swelling of the belly and to suppress the deterioration of the wearer's feeling of wearing.

In addition, at least a part of the outer side edges of the first low contraction region LER1 may be located at outer side of the outer side edge of the absorbent main body 10 in the width direction W. In the embodiment, a region that configures the outer side of the first low contraction region LER1 in the front-back direction L is located at outer side of the outer side edge of the absorbent main body 10 in the width direction W. Therefore, it is possible to dispose the first low contraction region LER1 in a region including the swelling of the belly even on the outer side of the outer side edge of the absorbent main body 10 in the width direction W. The first low contraction region LER1 corresponds to the shape of the belly, whereby it is possible to suppress excessive close attachment to the swelling of the belly and to suppress the deterioration of the wearer's feeling of wearing.

On the other hand, a region that configures the inner side of the first low contraction region LER1 in the front-back direction L may be located at inner side of the outer side edge of the absorbent main body 10 in the width direction W. Therefore, the first low contraction region LER1 may have an inverted trapezoidal shape in which the upper side (that is, the outer end edge of the first low contraction region LER1 in the front-back direction L) is longer than the lower side (that is, the inner end edge of the first low contraction region LER1 in the front-back direction L). In this case, the absorbent main body 10 that overlaps the first front contraction regions ER1 is pulled towards outer side in the width direction W by the contraction of a part of the first front contraction regions ER1. As a result, since it is possible to closely attach the absorbent main body 10 to the wearer's body, the leakage of excrement can be suppressed.

In addition, the length in the width direction of the outer end edge of the first low contraction region LER1 in the front-back direction L may be twice or more the length in the width direction W of the inner end edge of the first low contraction region LER1 in the front-back direction L. When the length in the width direction W of the outer end edge of the first low contraction region LER1 is set to twice or more the length in the width direction W of the inner end edge of the first low contraction region LER1, it becomes easy to cause the first low contraction region LER1 to correspond to the shape of the swelling of the belly, which broadens towards outer side in the width direction, in the upper side of the belly portion. The first low contraction region LER1 corresponds to the shape of the belly, whereby it is possible to suppress excessive close attachment to the swelling of the belly and to suppress the deterioration of the wearer's feeling of wearing.

The maximum length in the width direction W of the first low contraction region LER1 may be longer than the maximum length in the width direction W of the absorbent main body 10. In the first low contraction region LER1, since the contractive force of the front elastic member 60 is reduced, it is possible to suppress the contraction of the absorbent main body 10 in the width direction W. It is possible to closely attach the absorbent main body 10 to the body broadly, and the leakage of excrement can be suppressed.

On the outer side in the front-back direction L of the outer side edge of the absorbent article 1 in the front-back direction L, the length in the width direction W of the first low contraction region LER1 may be 1/2 or more of the length in the width direction W of the front exterior body 50A (front waistline region S1) as in the present embodiment.

As illustrated in Fig. 2, the first low contraction region LER1 may be disposed to straddle the outer end edge of the absorbent main body 10 in the front-back direction. Therefore, (a part of) the first low contraction region LER1 may be disposed to overlap the absorbent main body 10 in the thickness direction T. As illustrated in Fig. 7 and Fig. 8, the swelling of the belly continues from the recess at the lower end of the belly portion toward the upper side (outer side in the front-back direction). Therefore, when the wearer wears the absorbent article 1, the swelling of the belly is also present on the upper side (outer side in the front-back direction L) of the absorbent main body 10. Since the first low contraction region LER1 is disposed to straddle the outer end edge of the absorbent main body 10, it is possible to dispose the first low contraction region LER1 in the region corresponding to the shape of the swelling of the belly even on the upper side of the absorbent main body 10. The first low contraction region LER1 corresponds to the shape of the belly, whereby it is possible to suppress the excessive close attachment of the front exterior body 50A to the swelling of the belly and to suppress the deterioration of the wearer's feeling of wearing. In other embodiments, the first low contraction region LER1 may be disposed so as not to overlap the absorbent main body 10 in the thickness direction T. In addition, (a part of) the first low contraction region LER1 may be disposed to overlap the absorbent core 20 in the thickness direction T (that is, in a region that overlaps the absorbent core 20). In the first low contraction region LER1, since the contractive force of the front elastic member 60 is reduced, it is possible to suppress the shrinkage of the absorbent core 20 in the width direction W and to secure the absorption area of the absorbent core 20. Therefore, the leakage of excrement can be suppressed. In addition, in a region where the first low contraction region LER1 overlaps the absorbent core 20, the minimum length in the width direction W of the first low contraction region LER1 may be shorter than the length in the width direction W of the absorbent core 20. In this case, on the outer side in the width direction W of the minimum length in the width direction W of the first low contraction region LER1, the first front contraction regions ER1 that are located at outer side of the first low contraction region LER1 in the width direction W overlap the absorbent core 20 in the thickness direction T. Therefore, it is possible to closely attach the center of the absorbent core 20 in the width direction W to the body broadly with the first low contraction regions LER1 while closely attaching the end portions of the absorbent core 20 in the width direction W to the body by the contraction of the first front contraction regions ER1, and the leakage of excrement can be suppressed. It should be noted that, in other embodiments, the first low contraction region LER1 may be disposed so as not to overlap the absorbent core 20 in the thickness direction T.

The length of the first low contraction region LER1 in the front-back direction L may be 50% or more of the length of the side joining portion 80 in the front-back direction L. Since the first low contraction region LER1 is 50% or more of the length of the side joining portion 80 in the front-back direction, it is possible to broadly dispose the first low contraction region LER1 in the front-back direction L at the location corresponding to the shape of the swelling of the belly. Therefore, it is possible to suppress the deterioration of the wearer's feeling of wearing.

In the front-back direction L and on the inner side of the outer regions OR in the width direction W, the first low contraction region LER1 may be disposed in a region of 0% to 60% of the length of the side joining portion 80 in the front-back direction L (hereinafter, 0-60% region) starting from the outer end edge of the side joining portion 80 in the front-back direction L. On the inner side of the outer regions OR in the width direction W, the 0-60% region generally corresponds to the swelling of the wearer's belly. Therefore, the first low contraction region LER1 corresponds to the shape of the belly, whereby it is possible to suppress excessive close attachment to the swelling of the belly and to further suppress the deterioration of the wearer's feeling of wearing.

In the outer regions OR, the first low contraction region LER1 may be disposed in a region of 0% to 40% of the length of the side joining portion 80 in the front-back direction L (hereinafter, 0-40% region) starting from the outer end edge of the side joining portion 80 in the front-back direction L. In the outer regions OR, the 0-40% region generally corresponds to the swelling of the wearer's belly. Therefore, the first low contraction region LER1 corresponds to the shape of the belly, whereby it is possible to suppress excessive close attachment to the swelling of the belly and to further suppress the deterioration of the wearer's feeling of wearing. On the other hand, in the outer regions OR, the first front contraction regions ER1 may be disposed in a region of 40% to 60% of the length of the side joining portion 80 in the front-back direction L (40-60% region) starting from the outer end edge of the side joining portion 80 in the front-back direction L. The 40-60% region is a portion in which the shape of the swelling of the belly does not easily change due to a change in the wearer's action (for example, a change from the standing posture to the sitting posture (from the sitting posture to the standing posture)). Therefore, the front exterior body 50A is closely attached to the body in the 40-60% region due to the first front contraction regions ER1, whereby the front exterior body 50A does not easily slip down, and it is possible to continuously hold the front exterior body 50A.

The first low contraction region LER1 may be disposed in a region that overlaps the stretching/contracting sheet 56 in the thickness direction T as described below. The stretching/contracting sheet 56 has a surface that faces the wearer's skin and is thus capable of compressing the wearer's skin with the surface. Therefore, it is possible to closely attach the first low contraction region LER1 to the body by the contraction of the stretching/contracting sheet 56 while suppressing the occurrence of local fastening and to suppress the slip-down of the front exterior body 50A from the first low contraction region LER1 in which the contractive force of the front elastic member 60 is reduced.

The central contraction region CER is a region having higher contractility than the first low contraction region LER1. The central contraction region CER is a region that contracts due to the central elastic member 65. Therefore, in the central contraction region CER, the central elastic member 65 is disposed. In the present embodiment, the central contraction region CER may have contractility due to the stretching/contracting sheet 56.

At least a part of the central contraction region CER is disposed at outer side of the absorbent main body 10 in the front-back direction L. Since the front exterior body 50A contracts by the contraction of the central contraction region CER, the rigidity of the central contraction region CER becomes higher than the rigidity of the first low contraction region LER1. At least a part of the central contraction region CER is disposed at outer side of the absorbent main body 10 in the front-back direction L, whereby it is possible to make the central contraction region CER, in which the front exterior body 50A does not easily curl due to the high rigidity, present in the central region CR that does not overlap the absorbent main body 10. Additionally, it is possible to closely attach the central contraction region CER to the body by the contraction of the central contraction region CER, and it becomes easy for the front exterior body 50A to continuously cover the wearer's body. As described above, the absorbent article 1 is capable of reducing the curling of the front exterior body 50A with the central contraction region CER while suppressing the wearer's feeling of wearing with the first low contraction region. Therefore, it becomes easy for the absorbent article 1 to continuously cover the wearer's body while suppressing the deterioration of feeling of wearing.

The central contraction region CER may be disposed to straddle a center line C1 that passes through the center CO of the absorbent core 20 in the width direction W and extends in the front-back direction L. The central contraction region CER is disposed apart from the first low contraction region LER1 in the width direction W. Therefore, the first low contraction region LER1 is present between the central contraction region CER and the first low contraction region LER1 in the width direction W.

Here, in a case where the central contraction region CER is not disposed in the central region CR and only the first low contraction region LER1 is present, a difference in rigidity is generated with the outer end edge of the absorbent main body as a boundary by the degree of the rigidity of the absorbent main body. Since a force is likely to concentrate at a place having a difference in rigidity, (the first low contraction region LER1 of) the exterior body 50 is likely to bend from the outer end edge of the absorbent main body 10 having a large difference in rigidity as a base point. There is a possibility that the exterior body 50 may bend toward the skin surface side of the absorbent main body 10 to cause inside bending or may bend toward the non-skin surface side of the absorbent main body 10 to cause outside bending. The occurrence of inside bending or outside bending reduces a region where the exterior body 50 covers the wearer's skin or deteriorates the feeling of wearing. Therefore, the central contraction region CER may be disposed to straddle the outer end edge of the absorbent main body 10 in the front-back direction L in the thickness direction T. Therefore, the central contraction region CER may be disposed to overlap the absorbent main body 10 in the thickness direction T. Since it is possible to increase rigidity in the periphery of the outer end edge of the absorbent main body 10 in the front-back direction L with the central contraction region CER having high rigidity, it is possible to suppress the bending of (the first low contraction region LER1 of) the exterior body 50 from the outer end edge of the absorbent main body 10 in the front-back direction L as a base point. As a result, it is possible to reduce the curling of the exterior body 50 and to continuously cover the wearer's body while suppressing the deterioration of the feeling of wearing.

The central contraction region CER may be disposed in a region that does not overlap the absorbent core 20 in the thickness direction T. Even when the central contraction region CER contracts, it is possible to suppress the simultaneous contraction of the absorbent core 20. Therefore, it is possible to secure the absorption area of the absorbent core 20 and to further suppress the leakage of excrement.

The length of the central contraction region CER in the width direction W may be longer than 1/4 of the length of the absorbent core 20 in the width direction W. In such a case, it is easy to obtain an effect of securing the rigidity of the central region CR, and it is possible to reduce the curling of the exterior body 50.

Here, in the outer side portion of the exterior body 50 located at outer side of the outer side edge of the absorbent main body 10 in the width direction W, the rigidity does not increase due to the absorbent main body 10. Therefore, when the central contraction region CER is disposed in the outer side portion of the exterior body 50, the outer side portion of the exterior body 50 shrinks more than necessary, and thus there is a possibility that it may become easy for the wearer to feel the tightening by the exterior body 50, which may deteriorate the feeling of wearing. Therefore, the outer side edge of the central contraction region CER in the width direction W may be located at inner side of the outer side edge of the absorbent main body 10 in the width direction W. In such a case, the central contraction region CER is not disposed in the outer side portion of the front exterior body 50A, and it is possible to suppress the deterioration of the feeling of wearing.

The central contraction region CER may extend in the front-back direction L or may reach the outer end portion of the front exterior body 50A (front waistline region S1) in the front-back direction L. In such a case, due to the presence of the central contraction region CER having high rigidity, it is possible to suppress the curling of the front exterior body 50A from the outer end portion of the front exterior body 50A in the front-back direction L.

Alternatively, the central contraction region CER may be disposed only at inner side in a region from the outer end edge of the side joining portion 80 in the front-back direction L to 20% of the length of the side joining portion 80 in the front-back direction L (hereinafter, the upper part of the central region CR) in the front-back direction L. Therefore, the central contraction region CER may not be disposed in the upper part of the central region CR. The length of the upper part of the central region CR in the front-back direction L may be, for example, a 10 mm to 15 mm-long region from the outer end edge of the side joining portion 80 in the front-back direction L. Since the central contraction region CER is not disposed on the upper part of the central region CR, it is possible to keep the rigidity of the upper part of the central region CR low and to suppress the deterioration of the wearer's texture. Particularly, in the front waistline region S1, the upper part of the central region CR is a region that corresponds to the wearer's navel. Since the periphery of the wearer's navel is a sensitive portion, there is a possibility that the presence of a portion having high rigidity may cause the wearer to feel discomfort in the skin. When the rigidity is kept low in the upper part of the central region CR, the wearer does not feel any discomfort in the skin, and it is possible to suppress the deterioration of the feeling of wearing.

The elongation ratio of the central elastic member 65 that is disposed in the central contraction region CER may be twice or more. The contraction of the central elastic member 65 collects the members that configure the front exterior body 50A (for example, the front exterior sheet 52A, the front side top sheet 54A, and the stretching/contracting sheet 56), and the rigidity of the central contraction region CER, which will be described below, becomes high. As a result, it is possible to make it difficult for the front exterior body 50A to curl.

The elongation ratio of the central elastic member 65 may be measured by a well-known method. For example, the elongation ratio can be measured by the following method. First, in the absorbent article 1 in a natural state (for example, a disposable diaper), the length HO in the width direction W of an effective length portion that exhibits contractility (that is, a portion fixed to the front exterior sheet 52A or the like in a stretched state) in the central elastic member 65 of the measurement object is measured. Next, the length H1 in the width direction W of the effective length portion of the central elastic member 65 is measured in a stretched state in which the absorbent article 1 is stretched to the maximum extent in the width direction W such that wrinkles generated in the absorbent article 1 become invisible. In addition, the elongation ratio in the width direction W can be calculated with "elongation ratio = H1/H0".

The second front contraction regions ER2 have contractility due to the front elastic member 60 (second front elastic member 62). In the present embodiment, the second front contraction regions ER2 are regions that contract due to the second front elastic members 621 to 624. Therefore, in the second front contraction regions ER2, the second front elastic members 621 to 624 are disposed. In addition, in the present embodiment, the second front contraction regions ER2 may have contractility due to the stretching/contracting sheet 56.

The second front contraction regions ER2 are two contraction regions that extend towards inner side in the width direction W from both side portions of the front waistline region S1 in the width direction W. The second front contraction regions ER2 are disposed at outer side of the second low contraction region LER2 in the width direction W. In addition, in the present embodiment, the second front contraction regions ER2 are located at inner side of the front continuous contraction region FCR in the front-back direction L. Therefore, the second front contraction regions ER2 are disposed at inner side of the first front contraction regions ER1 in the front-back direction L.

In the present embodiment, since the first front contraction regions ER1 are located at outer side of the first low contraction region LER1 in the width direction W, and the second front contraction regions ER2 are located at outer side of the second low contraction region LER2 in the width direction W, it is easy to dispose the first front contraction regions ER1 and the second front contraction regions ER2 on the side portions of the belly portion in the width direction. In the side portion of the belly portion in the width direction, it is difficult for the belly to swell compared with the center of the belly portion in the width direction. In the side portions where the belly does not easily swell, the front exterior body 50A is closely attached to the body with the first front contraction regions ER1 and the second front contraction regions ER2, whereby it is possible to continuously hold the front exterior body 50A while suppressing the deterioration of the wearer's feeling of wearing. The front exterior body 50A does not slip down, and it is possible to continuously hold the absorbent article 1 on the body.

The front continuous contraction region FCR is a region that extends from one side portion to the other side portion of the front exterior body 50A (front waistline region S1) in the width direction W. The front continuous contraction region FCR has contractility due to the front elastic member 60 (third front elastic member 63). Specifically, the front continuous contraction region FCR is a region that contracts due to the third front elastic members 631 and 632 and is a region in which the third front elastic members 631 and 632 are disposed. In addition, in the present embodiment, the front continuous contraction region FCR has contractility due to the stretching/contracting sheet 56.

The front continuous contraction region FCR is disposed between the first front contraction regions ER1 and the second front contraction regions ER2 in the front-back direction L. In addition, the front continuous contraction region FCR is disposed between the first low contraction region LER1 and the second low contraction region LER2 in the front-back direction L.

The outer end edge of the front continuous contraction region FCR in the front-back direction L is defined by the first front elastic member 61 that is disposed in the first front contraction region ER1. Specifically, the outer end edge of the front continuous contraction region FCR in the front-back direction L is a region that is on the inner side of the first front elastic member 619 which is located on the innermost side in the front-back direction L as the first front elastic member 61. Therefore, the front continuous contraction region FCR is also located at outer side of the third front elastic member 631 in the front-back direction L.

Similarly, the inner end edge of the front continuous contraction region FCR in the front-back direction L is defined by the second front elastic member 62 that is disposed in the second front contraction region ER2. Specifically, the outer end edge of the front continuous contraction region FCR in the front-back direction L is a region that is on the outer side of the second front elastic member 621 which is located on the outermost side in the front-back direction L as the second front elastic member 62. Therefore, the front continuous contraction region FCR is also located at inner side of the third front elastic member 632 in the front-back direction L.

In the front-back direction L, the front continuous contraction region FCR may be disposed in a region that is 60% to 80% of the length of the side joining portion 80 in the front-back direction L starting from the outer end edge of the side joining portion 80 in the front-back direction L. The region that is 60% to 80% of the length of the side joining portion 80 in the front-back direction L starting from the outer end edge of the side joining portion 80 generally corresponds to the recess at the lower end of the wearer's belly portion. The front continuous contraction region FCR is disposed in the region, whereby it is possible to cause the front continuous contraction region FCR to correspond to the recess at the lower end of the belly portion, and the front continuous contraction region FCR does not easily slip down toward the crotch side. As a result, it is possible to suppress the slip-down of the front exterior body 50A toward the crotch side, and it becomes easy for the front exterior body 50A to continuously cover the wearer's body. In addition, when the recess at the lower end of the belly portion is caused to correspond to the front continuous contraction region FCR, it is possible to cause a portion where the belly swells on the upper side (outer side in the front-back direction) of the recess at the lower end of the belly portion to correspond to the first low contraction region LER1. Therefore, it is possible to suppress the deterioration of the wearer's feeling of wearing without closely attaching the absorbent article 1 excessively to the portion where the belly swells on the upper side of the recess at the lower end of the belly portion.

It should be noted that, in the case of, for example, an M-sized absorbent article 1, the front continuous contraction region FCR may be disposed in a 50 mm to 65 mm-long region from the outer end edge of the side joining portion 80 in the front-back direction L. In the case of, for example, an L-sized absorbent article 1, the front continuous contraction region FCR may be disposed in a 60 mm to 75 mm-long region from the outer end edge of the side joining portion 80 in the front-back direction L.

As illustrated in Fig. 2, the front continuous contraction region FCR may be disposed in a region that overlaps the absorbent core 20 in the thickness direction T. Since the absorbent core 20 can be closely attached to the wearer's body by the contraction of the front continuous contraction region FCR, the leakage of excrement can be suppressed.

The absorbent core 20 may have a low-basis-weight portion having a lower basis weight than the periphery in a region that overlaps the front continuous contraction region FCR in the thickness direction T. In such a case, in the low-basis-weight portion that overlaps the front continuous contraction region FCR, the thickness of the absorbent core 20 is likely to become thin. As the thickness of the absorbent core 20 becomes thinner, it becomes easier to closely attach the front exterior body 50A to the wearer's body due to the contractive force of the front continuous contraction region FCR. Therefore, it becomes easy for the absorbent article 1 to continuously cover the wearer's body. Furthermore, in a case where the absorbent core 20 contains a water absorptive polymer, the basis weight of the water absorptive polymer is also likely to become low in the low-basis-weight portion. Therefore, in the low-basis-weight portion, the absorbent core does not easily expand due to the absorption of excrement by the water absorptive polymer, and it is possible to reduce an increase in the thickness of the absorbent core 20. Even after excretion, it becomes easy to closely attach the front exterior body 50A to the wearer's body, and it becomes easy for the absorbent article 1 to continuously cover the wearer's body. Therefore, the basis weight of the water absorptive polymer in the low-basis-weight portion may be lower than the basis weight of the water absorptive polymer in the absorbent core 20 in a region different from the low-basis-weight portion (for example, the region that overlaps the second low contraction region LER2 in the thickness direction T). In addition, the basis weight of pulp in the low-basis-weight portion may be lower than the basis weight of the pulp in the absorbent core 20 in a region different from the low-basis-weight portion (for example, the region that overlaps the second low contraction region LER2 in the thickness direction T).

The second low contraction region LER2 is a region in which the contractive force is reduced compared with the contractive force of the second front contraction region ER2. Here, the contractive force of the second low contraction region LER2 can be reduced in the same manner as that of the first low contraction region LER1. In the first low contraction region LER1 and the second low contraction region LER2, since the contractive force is reduced compared with the contractive force in the contraction regions, it is possible to reduce the strong fastening of the belly compared with the fastening in the contraction regions. Therefore, it is possible to dispose the front exterior body 50A that is disposed in the front waistline region S1 without closely attaching the front exterior body 50A excessively to the belly, which can suppress the deterioration of the wearer's feeling of wearing.

In addition, the low contraction regions (the first low contraction region LER1 and the second low contraction region LER2) may be disposed to straddle the center of the exterior body in the width direction. In this case, the first low contraction region LER1 and the second low contraction region LER2 are disposed at the center of the wearer's belly portion in the width direction. Regarding the shape of the wearer's belly portion at the center in the width direction, generally, the belly swells from the recess at the lower end of the belly portion toward the upper side (outer side in the front-back direction L). The low contraction regions (the first low contraction region LER1 and the second low contraction region LER2) are disposed against the swelling of the belly, whereby it is possible to suppress the excessive close attachment of the front exterior body 50A to the swelling of the belly and to suppress the deterioration of the wearer's feeling of wearing.

The second low contraction region LER2 is sandwiched between the two second front contraction regions ER2 in the width direction W. The second low contraction region LER2 is located at inner side of the front continuous contraction region FCR in the front-back direction L. Therefore, the second low contraction region LER2 is disposed at inner side of the first low contraction region LER1 in the front-back direction L. With such a disposition, it is possible to more closely attach the absorbent main body 10 to the body by the contraction of the front continuous contraction region FCR, and the leakage of excrement can be suppressed. Meanwhile, the second low contraction region LER2 is disposed at inner side of the front continuous contraction region FCR in the front-back direction L, whereby it is possible to flexibly attach the absorbent main body 10 to the body in a broad region even on the crotch region S3 side, and the leakage of excrement can be suppressed.

The maximum length in the width direction W of the second low contraction region LER2 may be shorter than the maximum length in the width direction W of the first low contraction region LER1. That is, the maximum length in the width direction W of the first low contraction region LER1 may be longer than the maximum length in the width direction W of the second low contraction region LER2. In addition, the area of the first low contraction region LER1 may be larger than the area of the second low contraction region LER2. As illustrated in Fig. 6, compared with the lower side of the belly portion, on the upper side of the belly portion, the area is broad, and the length in the width direction W is also long. Therefore, the first low contraction region LER1 having a large area and a long maximum length in the width direction W is disposed on the upper side of the belly portion, and the second low contraction region LER2 having a small area and a short maximum length in the width direction W is disposed on the lower side of the belly portion, whereby it is possible to dispose the first low contraction region LER1 and the second low contraction region LER2 in a region that corresponds to the shape of the swelling of the belly. The first low contraction region LER1 and the second low contraction region LER2 correspond to the shape of the belly, whereby it is possible to suppress excessive close attachment to the swelling of the belly and to suppress the deterioration of the wearer's feeling of wearing.

It should be noted that the central region CR may have the second low contraction region LER2. The central region CR may include the first low contraction region LER1, the second low contraction region LER2, and the central contraction region CER.

As illustrated in Fig. 2 to Fig. 5, in the present embodiment, the absorbent article 1 has a sheet region SR. The sheet region SR has contractility in the width direction W due to at least the stretching/contracting sheet 56. The sheet region SR has a high contraction sheet region HSR and a low contraction sheet region LSR. The high contraction sheet region HSR is a region in the sheet region SR in which the elastic member (front elastic member 60) is disposed to overlap the stretching/contracting sheet 56 in the thickness direction. The low contraction sheet region LSR is a region in the sheet region SR in which the contractive force is reduced compared with the contractive force in the high contraction sheet region HSR and the contractility is lower than the contractility of the high contraction sheet region HSR.

In the high contraction sheet region HSR, the front elastic member 60 is disposed to overlap the stretching/contracting sheet 56, whereby it is possible to increase the contractive force. On the other hand, in the low contraction sheet region LSR, the contractive force of the front elastic member 60 is reduced, and the contractive force is lower than the contractive force of the high contraction sheet region HSR. The low contraction sheet region LSR and the high contraction sheet region HSR are arranged in the width direction W, whereby it is possible to change compression in the width direction W. Therefore, it is possible to increase compression in a region where it is necessary to closely attach the absorbent article 1 to the body and to decrease compression in a region where it is necessary to cover the body with the absorbent article 1 along the shape. As a result, it is possible to hold the absorbent article 1 on the body and to suppress the deterioration of the wearer's feeling of wearing. In addition, since the thread-like or band-like front elastic member 60 is easier to control during the manufacturing of the absorbent article than the stretching/contracting sheet 56, it is possible to change the compression in the sheet region SR in the width direction W without enhancing the production difficulty. Therefore, it is possible to hold the absorbent article 1 on the body while suppressing an increase in production costs and to suppress the deterioration of the wearer's feeling of wearing.

In the present embodiment, the high contraction sheet region HSR corresponds to the first front contraction regions ER1, the second front contraction regions ER2, and the central contraction region CER. The low contraction sheet region LSR corresponds to the first low contraction region LER1 and (a part of) the second low contraction region LER2.

The low contraction sheet region LSR may be disposed between the high contraction sheet regions HSR in the width direction W. In such a case, on both sides of the low contraction sheet region LSR in the width direction W, it is possible to closely attach the high contraction sheet regions HSR to the body by the contraction of the high contraction sheet regions HSR. Therefore, it is possible to make it difficult for the low contraction sheet region LSR that is disposed between the high contraction sheet regions HSR to separate from the body. Therefore, in the low contraction sheet region LSR, it is possible to hold the absorbent article 1 on the body along the shape of the body. It should be noted that, in the present embodiment, as illustrated in Fig. 2, the first low contraction region LER1 is disposed between the first front contraction regions ER1 in the width direction W. In addition, the first low contraction region LER1 is disposed between the first front contraction region ER1 and the central contraction region CER.

During the manufacturing of the absorbent article 1, there is a case where a continuous sheet member 100c is conveyed in a conveyance direction MD as illustrated in Fig. 9A. The continuous sheet member 100c is composed of a continuous sheet (continuous body) constituting the exterior body 50. For example, the continuous sheet member 100c has a continuous body of the exterior sheet 52, a continuous body 54c of the side top sheet 54, and a continuous body 56c of the stretching/contracting sheet 56. Here, there is a case where the continuous sheet member 100c is conveyed in a state of being stretched in the conveyance direction MD such that wrinkles are not formed in the continuous sheet member 100c. Due to stretching at this time (that is, a force F that stretches the continuous sheet member 100c), the continuous sheet member 100c, the width of the continuous sheet member 100c in an orthogonal direction CD that is orthogonal to the conveyance direction MD becomes small (that is, neck-in) in some cases, and there is a case where it is not possible to stably convey the continuous sheet member 100c.

Therefore, the sheet region SR may have a first region in which the high contraction sheet region HSR and the low contraction sheet region LSR are arranged in the conveyance direction MD (that is, the width direction W) (for example, a region of the first front contraction regions ER1 and the first low contraction region LER1), and a second region in which only the high contraction sheet region HSR is disposed in the width direction W (that is, the front continuous contraction region FCR) as illustrated in Fig. 9B. In addition, the second region and at least the low contraction sheet region LSR in the first region may be disposed side by side in the orthogonal direction CD (that is, the front-back direction L). Therefore, the low contraction sheet region LSR in the first region and the second region in which only the high contraction sheet region HSR is disposed are arranged in the orthogonal direction CD, whereby a region of the low contraction sheet region alone is no longer present in the orthogonal direction CD. That is, the high contraction sheet region HSR is continuously disposed in the conveyance direction MD. While the force F that stretches the continuous sheet member 100c acts in a direction in which the high contraction sheet region HSR is stretched, the contractive force f of the high contraction sheet region acts against the force F that stretches the continuous sheet member 100c. Since the contractive force f of the high contraction sheet regions acts along the conveyance direction MD at all times due to the high contraction sheet region HSR that is continuously disposed, the force F that stretches the continuous sheet member 100c is weakened, and it is possible to reduce neck-in. It becomes possible to stably convey the continuous sheet member 100c.

In the present embodiment, the absorbent article 1 has the front exterior body 50A having the sheet region SR. Therefore, it is possible to provide a region in which the front exterior body 50A is easily closely attached to the wearer's waistline and a region in which the front exterior body 50A is easily attached to the wearer's waistline along the shape, and it is possible to continuously hold the front exterior body 50A on the body while suppressing the deterioration of the wearer's feeling of wearing. The front exterior body 50A does not slip down, and it is possible to continuously hold the absorbent article 1 on the body.

As illustrated in Fig. 2, the low contraction sheet region LSR may be disposed to straddle the center of the front exterior body 50A in the width direction W. The high contraction sheet regions HSR may be respectively located at outer side of the low contraction sheet region LSR in the width direction W. Therefore, the low contraction sheet region LSR is disposed to straddle the center of the front exterior body 50A in the width direction W and is thus disposed at the center of the wearer's belly portion in the width direction W. As a result, it is possible to dispose the low contraction sheet region LSR against the swelling of the belly, and it is possible to suppress the excessive close attachment of the front exterior body 50A to the swelling of the belly and to suppress the deterioration of the wearer's feeling of wearing. In addition, contraction of the high contraction sheet regions HSR makes it possible to closely attach the absorbent article 1 to the body from the respective outer sides of the low contraction sheet region LSR in the width direction W with the high contraction sheet regions HSR. Therefore, it is possible to make it difficult for the low contraction sheet region LSR to separate from the body.

In addition, both outer side edges of the low contraction sheet region LSR may be located at outer side in the width direction W as the low contraction sheet region LSR extends outer side in the front-back direction L. In such a case, it is possible to dispose the low contraction sheet region LSR to correspond to the swelling of the belly. It is possible to suppress the excessive close attachment of the front exterior body 50A to the swelling of the belly and to suppress the deterioration of the wearer's feeling of wearing.

In addition, the length in the width direction W of the outer end edge of the low contraction sheet region LSR in the front-back direction L may be twice or more the length in the width direction of the inner end edge of the low contraction sheet region LSR in the front-back direction L. Regarding the shape of the wearer's belly portion at the center in the width direction, the belly swells from the recess at the lower end of the belly portion toward the upper side (outer side in the front-back direction L). Therefore, the length in the width direction W of the front waistline region S1 on the upper side of the belly portion is longer than the length in the width direction of the front waistline region S1 on the lower side of the belly portion. The length in the width direction W of the outer side edge of the low contraction sheet region LSR is set to twice or more the length in the width direction W of the inner end edge of the low contraction sheet region LSR, whereby it is possible to dispose the low contraction sheet region LSR to correspond to the swelling of the belly. It is possible to suppress the excessive close attachment of the front exterior body 50A to the swelling of the belly and to suppress the deterioration of the wearer's feeling of wearing.

In addition, at least a part of the outer side edges of the low contraction sheet region LSR in the width direction W may be located at outer side of the outer side edges of the absorbent main body 10 in the width direction W. Regarding the shape of the wearer's belly portion at the center in the width direction, the belly swells from the recess at the lower end of the belly portion toward the upper side (outer side in the front-back direction). Therefore, there are many cases where the swelling of the belly reaches outer side in the width direction W beyond the outer side edges of the absorbent main body 10. Therefore, at least a part of the outer side edge of the low contraction sheet region LSR is located at outer side of the outer side edges of the absorbent main body 10 in the width direction W, whereby it is possible to dispose the low contraction sheet region LSR in a region including the swelling of the belly that is located at outer side of the absorbent main body 10 in the width direction W. It is possible to suppress the excessive close attachment of the front exterior body 50A to the swelling of the belly and to suppress the deterioration of the wearer's feeling of wearing.

In addition, a part of the high contraction sheet regions HSR may overlap the absorbent main body 10 in the thickness direction T. It is possible to dispose the low contraction sheet region LSR against the swelling of the belly and to closely attach the absorbent main body 10 to the body by the contraction of the high contraction sheet regions HSR. Therefore, the leakage of the excrement can be suppressed. In addition, a part of the high contraction sheet regions HSR may overlap the absorbent core 20 in the thickness direction T. It is possible to dispose the low contraction sheet region LSR against the swelling of the belly and to closely attach the absorbent core 20 to the body by the contraction of the high contraction sheet regions HSR. Therefore, the leakage of the excrement can be suppressed.

In addition, one or more cuts may be formed in the stretching/contracting sheet 56 that configures the low contraction sheet region LSR. The cuts may penetrate the stretching/contracting sheet 56 or may not penetrate the stretching/contracting sheet 56. The cuts may be slits formed on the stretching/contracting sheet 56 or may be openings. Therefore, one or more cuts may be generated in the stretching/contracting sheet 56 in the first low contraction region LER1 and the second low contraction region LER2. Since the contractive force does not act in the stretching/contracting sheet portion in which one or more cuts are formed, it is possible to further reduce the contractive force of the stretching/contracting sheet 56 in the low contraction sheet region LSR. It is possible to attach the absorbent article 1 to the body along the shape more flexibly and to further suppress the deterioration of the wearer's feeling of wearing.

Here, as described in the first method described above, in a case where a low contraction region (for example, the first low contraction region LER1) is provided by the contraction of the cut front elastic member 60, the non-fixed portion of the front elastic member 60 may overlap the low contraction sheet region LSR in the thickness direction T. In addition, the non-fixed portion of the front elastic member 60 may be conveyed or the like during manufacturing to overlap the high contraction sheet region HSR. Therefore, the non-fixed portion of the front elastic member 60 may overlap the stretching/contracting sheet 56. Here, the non-fixed portion of the front elastic member 60 is a portion in which the front elastic member 60 contracts by cutting. Therefore, the low contraction sheet region LSR does not overlap the thread-like or band-like front elastic member 60 in a stretched state in the thickness direction T. Therefore, since the contractility in the low contraction sheet region LSR is determined only by the stretching/contracting sheet 56, it becomes easy to control the contractility in the low contraction sheet region LSR. In addition, the non-fixed portion is continuous with the fixed portion of the front elastic member 60 that is disposed (fixed) in the high contraction sheet region HSR and thus extends from the boundary between the low contraction sheet region LSR and the high contraction sheet region HSR. Therefore, in a case where the wearer is able to see the non-fixed portion, it becomes easy for the wearer to see the boundary of the low contraction sheet region LSR and to identify a region in which the front elastic member covers the body (belly) along the shape. Therefore, the wearer is likely to feel safe due to the presence of the non-fixed portion.

The elongation ratio of the stretching/contracting sheet 56 may be lower than the elongation ratio of the front elastic member 60 that overlaps the stretching/contracting sheet 56 in the thickness direction T. In such a case, it is possible to decrease the contractive force of the low contraction sheet region LSR that corresponds to the swelling of the belly. It is possible to suppress the excessive close attachment of the low contraction sheet region LSR to the swelling of the belly and to suppress the deterioration of the wearer's feeling of wearing.

Next, the region in the rear waistline region S2 (rear exterior body 50B) will be described. As illustrated in Fig. 2 and the like, the rear waistline region S2 (rear exterior body 50B) has a rear continuous contraction region RCR, a rear contraction region RER, and a rear low contraction region RLR.

The rear continuous contraction region RCR is a region that extends from one side portion to the other side portion of the rear exterior body 50B (rear waistline region S2) in the width direction W. In addition, the rear continuous contraction region RCR is adjacent to the front continuous contraction region FCR in the width direction W in a state in which the front exterior body 50A and the rear exterior body 50B are joined to each other. The rear continuous contraction region RCR has contractility due to the rear elastic member 70 (first rear elastic member 71). Here, the front continuous contraction region FCR and the rear continuous contraction region RCR are made adjacent to each other in the width direction W in a state in which the front exterior body 50A and the rear exterior body 50B are joined to each other, whereby it is possible to dispose a region that includes the front continuous contraction region FCR and the rear continuous contraction region RCR and has contractility in a manner such that the region fully covers the wearer's waistline when the wearer wears the absorbent article. It is possible to hold the absorbent article 1 on the body in this region that continuously and fully covers the wearer's waistline and to suppress the slip-down of the exterior body 50 toward the crotch side. Therefore, in the front waistline region S1, it is possible to suppress the deterioration of the wearer's feeling of wearing with the first low contraction region LER1 and the second low contraction region LER2 and to facilitate the front continuous contraction region FCR and the rear continuous contraction region RCR to continuously cover the wearer's body.

In the present embodiment, the rear continuous contraction region RCR is a region having contractility due to the first rear elastic members 711 to 717 and the first rear elastic members 71a to 71c. Therefore, the first rear elastic member 71 is disposed in the rear continuous contraction region RCR. The rear continuous contraction region RCR has a first rear continuous contraction region RCR1 and a second rear continuous contraction region RCR2.

The first rear continuous contraction region RCR1 is a region that is not directly adjacent to the front continuous contraction region FCR in the width direction W in a state in which the front exterior body 50A and the rear exterior body 50B are joined to each other. The first rear continuous contraction region RCR1 includes the first rear elastic members 711 to 717. It should be noted that, in the present embodiment, the first rear continuous contraction region RCR1 is adjacent to the first front contraction regions ER1 in the width direction W. Therefore, the first rear elastic members 71 are disposed adjacent to each other in the width direction W, whereby it is possible to closely attach the absorbent article 1 to the body from both the stomach side and the back-side at the same location as the first front contraction regions ER1 in the front-back direction L.

The second rear continuous contraction region RCR2 is a region that is directly adjacent to the front continuous contraction region FCR in the width direction W in a state in which the front exterior body 50A and the rear exterior body 50B are joined to each other. The second rear continuous contraction region RCR2 includes the first rear elastic members 71a to 71c. The second rear continuous contraction region RCR2 is disposed at inner side of the first rear continuous contraction region RCR1 in the front-back direction L. It should be noted that, since the second rear continuous contraction region RCR2 is adjacent to the front continuous contraction region FCR, the rear continuous contraction region RCR is adjacent to the front continuous contraction region FCR.

Here, the elongation ratio of the third front elastic member 63 that is disposed in the front continuous contraction region FCR may be higher than the elongation ratio of the first rear elastic member 71 that is disposed in the rear continuous contraction region RCR. Here, the recess of the wearer's belly portion is directed to the upper side as the recess at the lower end of the belly portion extends from the center in the width direction toward the side portion in the width direction W. On the other hand, the recess in the wearer's back is located on the upper side of the recess at the center of the belly portion in the width direction W and is substantially continuous with the recess on the side portion of the belly portion. The recess in the wearer's back is located on the upper side of the swelling of the buttocks. Therefore, the rear continuous contraction region RCR is located on the upper side of the swelling of the buttocks, whereby the rear continuous contraction region RCR does not easily slip down toward the crotch side due to the swelling of the buttocks (refer to Fig. 7). Therefore, when the slip-down of the front continuous contraction region FCR toward the crotch side is suppressed by increasing the elongation ratio of the third front elastic member 63, it is possible to suppress the slip-down of the region that continuously and fully covers the wearer's waistline toward the crotch side in a well-balanced manner and to facilitate the absorbent article 1 to continuously cover the wearer's body. The elongation ratio can be measured by the above-described method.

In addition, the contractive force of the first front elastic member 61 that is disposed in the first front contraction region ER1 may be larger than the contractive force of the first rear elastic member 71 that is disposed in the rear continuous contraction region RCR adjacent to the first front contraction region ER1. The first rear elastic member 71 is disposed adjacent to the first front contraction region ER1 in the width direction W, whereby it is possible to closely attach the absorbent article 1 to the body from both the stomach side and the back-side at the same location as the first front contraction region ER1 in the front-back direction L. Since the first front contraction regions ER1 are disposed at outer side of the first low contraction region LER1 in the width direction W, it is possible to suppress the slip-down of the front exterior body 50A from the front waistline region S1 by setting the contractive force to be larger than the contractive force of the first rear elastic member 71 on the back-side.

It should be noted that the contractive force of the elastic member can be measured by, for example, the following method. The length H0 in the width direction W of the effective length portion of the elastic member is measured in a stretched state in which the absorbent article 1 is stretched to the maximum extent in the front-back direction L such that wrinkles generated in the absorbent article 1 become invisible. Next, the elastic member is removed from the absorbent article 1, and the length H1 in the width direction W of the effective length portion of the elastic member is measured. In addition, the contractive force of the elastic member can be calculated with "contractive force = H0/H1".

It should be noted that, instead of the contractive force, the yarn count that defines the thickness of the first front elastic member 61 may be set to be smaller than the yarn count of the first rear elastic member 71. The elongation ratio of the first front elastic member 61 may be set to be larger than the elongation ratio of the first rear elastic member 71.

In addition, the rear continuous contraction region RCR may be disposed in a region that does not overlap the absorbent core 20 in the thickness direction T. In such a case, it is possible to suppress a decrease in the contractive force by the rear continuous contraction region RCR due to the rigidity of the absorbent core 20. Therefore, the absorbent article 1 is closely attached to the body on the back-side, whereby it is possible to suppress the slip-down of the front exterior body 50A not only from the rear waistline region S2 but also from the front waistline region S1.

The rear contraction region RER is a region that contracts due to the second rear elastic members 721 to 724 and is a region in which the second rear elastic members 721 to 724 are disposed.

The rear contraction regions RER are two contraction regions that extend towards inner side in the width direction W from both side portions of the rear waistline region S2 in the width direction W. The rear contraction regions RER are disposed at outer side of the rear low contraction region RLR in the width direction W.

The rear low contraction region RLR is a region in which the contractive force is reduced compared with the contractive force of the rear contraction region RER. The contractive force of the rear low contraction region RLR can be reduced in the same manner as that of the first low contraction region LER1.

The rear low contraction region RLR is disposed between the two rear contraction regions RER in the width direction W. The rear low contraction region RLR is disposed at inner side of the rear continuous contraction region RCR in the front-back direction L.

It should be noted that the outer end edge of the front exterior body 50A in the front-back direction L (that is, a waist opening of the front exterior body 50A) may be closer to the center O of the absorbent main body 10 in the front-back direction than the outer end edge of the rear exterior body 50B in the front-back direction L (that is, a waist opening of the rear exterior body 50B). That is, the absorbent main body 10 may be closer to the front exterior body 50A side. In this case, in the front waistline region S1, the distance from the outer end edge of the absorbent main body 10 in the front-back direction L to the waist opening becomes short. Therefore, in a case where the front exterior body 50A curls, the distance becomes shorter, and excrement is likely to leak from the waist opening of the front exterior body. Therefore, when the central contraction region CER is disposed in the front waistline region S1, the front exterior body 50A does not easily curl, and it is possible to reduce the leakage of excrement from the waist opening. It should be noted that, in other embodiments, the same effect can be obtained even in a case where the central contraction region CER is present in the rear waistline region S2.

In other embodiments, the outer end edge of the front exterior body 50A in the front-back direction L (that is, the waist opening of the front exterior body 50A) may be farther from the center of the absorbent main body 10 in the front-back direction than the outer end edge of the rear exterior body 50B in the front-back direction L (that is, the waist opening of the rear exterior body 50B). That is, the absorbent main body 10 may be closer to the rear exterior body 50B side. Therefore, in the front waistline region S1, the distance from the outer end edge of the absorbent main body 10 in the front-back direction to the waist opening becomes long. As the distance increases, the area in which the front exterior body 50A is capable of curling increases. Therefore, when the central contraction region CER is disposed in the front waistline region S1, the front exterior body 50A does not easily curl, and it is possible to reduce the area in which the front exterior body 50A curls. It should be noted that, in other embodiments, the same effect can be obtained even in a case where the central contraction region CER is present in the rear waistline region S2.

It should be noted that the center O of the absorbent main body 10 in the front-back direction is the midpoint in a case where the absorbent main body 10 is equally divided into two portions in the front-back direction L. The closeness to the center O of the absorbent main body 10 can be measured by comparing the shortest distance from the midpoint to the outer end edge of the front exterior body 50A in the front-back direction L and the shortest distance from the midpoint to the outer end edge of the rear exterior body 50B in the front-back direction L.

### (3) OTHER EMBODIMENTS

Hitherto, the present invention has been described in detail using the above-described embodiment, but it is apparent to those skilled in the art that the present invention is not limited to the embodiment described in the present specification. The present invention can be carried out as a correction and modification aspect without departing from the gist and scope of the present invention that is defined by the description of claims. Therefore, the description of the present specification is intended for exemplary description and does not have any limiting meaning on the present invention.

In the above-described embodiment, the waistline region having the central region CR including the first low contraction region LER1 and the central contraction region CER is the front waistline region S1. Generally, the wearer's belly bulges on the upper side of the navel and recedes in the lower belly portion (particularly, in the narrow part of the lower belly portion) on the lower side of the navel. Therefore, the length of the waistline of the belly decreases from the waist opening that is disposed on the upper side of the navel toward the crotch side, and thus the front exterior body 50A that is disposed on the stomach side is more likely to curl than the rear exterior body 50B that is disposed on the back-side. The waistline region having the central region CR is the front waistline region, and the front exterior body 50A having the central contraction region CER is disposed on the stomach side, whereby it is possible to reduce the curling of the front exterior body 50A on the stomach side, which is likely to curl.

In addition, the waistline region having the central region CR may be the rear waistline region S2. In this case, there is a concern that, when the wearer pulls up the absorbent article 1 to wear the absorbent article 1, the rear exterior body 50B may be caught by the bulge of the buttocks or the rear exterior body 50B may curl. However, since the rigidity of the central region CR increases due to the presence of the central contraction region CER, it is possible to make the curling of the rear exterior body 50B difficult even when the rear exterior body 50B is caught by the bulge of the buttocks.

In the above description, a part of the central contraction region CER is disposed at outer side of the absorbent main body 10 in the front-back direction L, but the configuration is not limited thereto. All of the central contraction region CER may be disposed at outer side of the absorbent main body 10 in the front-back direction L.

In the above description, a region in which the front elastic member 60 is not disposed (for example, a region in which the contractive force by the front elastic member 60 is zero) may be disposed at outer side of the central region CR (first low contraction region LER1) in the front-back direction L. The region may reach the outer end portion of the front exterior body 50A (front waistline region S1) in the front-back direction L.

In the above description, the central contraction region CER is disposed in the absorbent article 1, but the configuration is not limited thereto. The central contraction region CER may not be disposed. In such a case, it is possible to secure the first low contraction region LER1 in a broad region.

In the above description, the front continuous contraction region FCR is disposed in the absorbent article 1, but the configuration is not limited thereto. The front continuous contraction region FCR may not be provided. For example, the first low contraction region LER1 and the second low contraction region LER2 may be arranged continuously in the front-back direction. Thus, the front exterior body 50A is capable of covering the wearer's body further along the shape of the body, which can suppress the deterioration of the wearer's feeling of wearing.

In the above description, the outer side edges of the first low contraction region LER1 in the width direction W are disposed in the outer regions OR on the outer side of the absorbent main body 10 in the front-back direction L, but the configuration is not limited thereto. The outer side edges of the first low contraction region LER1 in the width direction W may be disposed at inner side of the outer regions OR in the width direction W on the outer side of the absorbent main body 10 in the front-back direction L. In such a case, it is possible to reduce the curling of the front exterior body 50A.

In the above-described embodiment, the lengths in the front-back direction L of the outer end edge of the side joining portion 80 in the front-back direction L and the outer end edge of the front exterior body 50A in the front-back direction L (that is, the waist opening) coincide. Therefore, the region of the length in the front-back direction L of the side joining portion 80 (for example, the 0-60% region or the like) is defined starting from the outer end edge of the side joining portion 80 in the front-back direction L, but the configuration is not limited thereto. In a case where the outer end edge of the front exterior body 50A in the front-back direction L is present at outer side of the outer end edge of the side joining portion 80 in the front-back direction L in the front-back direction L, the region may be defined starting from the outer end edge of the front exterior body 50A in the front-back direction L. In this case, the 0-60% region, the 0-40% region, or the like may be defined using the length from the outer end edge of the front exterior body 50A in the front-back direction L to the inner end edge of the side joining portion 80 in the front-back direction L as a reference.

In the above-described embodiment, the length from the center of the front exterior body 50A in the width direction W to the outer side edge of the first low contraction region LER1 in the width direction W (hereinafter, the width length of the first low contraction region LER1) is maximized on the outermost side of the first low contraction region LER1 in the front-back direction L, but the configuration is not limited thereto. The width length of the first low contraction region LER1 may be maximized in any location between the outermost edge of the first low contraction region LER1 in the front-back direction L and the outer side edge of the absorbent main body 10 in the front-back direction L. Therefore, the width length of the first low contraction region LER1 on the outermost side of the first low contraction region LER1 in the front-back direction L may be shorter than the maximum width length of the first low contraction region LER1. In this case, the length in the width direction W of the first front elastic member 61 that is located at outer side in the front-back direction L may be shorter than the length in the width direction W of the first front elastic member 61 that configures the maximum width length of the first low contraction region LER1.

In the above-described embodiment, the absorbent article 1 has been described as an example, but is not limited thereto. The above-described configuration can be applied to the sheet member having the stretching/contracting sheet 56 and the thread-like or band-like elastic member. Further, the stretching/contracting sheet 56 has been disposed in the front waistline region S1, but is not limited thereto. The stretching/contracting sheet 56 may be disposed in the rear waistline region S2. In the rear waistline region S2, the high contraction sheet region HSR and the low contraction sheet region LSR may be disposed similar to the front waistline region S1.

### [Reference Signs List]

1 : Absorbent article
10 : Absorbent main body
15 : Leg opening
20 : Absorbent core
30 : Top-surface sheet
40 : Back-surface sheet
50 : Exterior body
50A : Front exterior body
50B : Rear exterior body
52 : Exterior sheet
52A : Front exterior sheet
52B : Rear exterior sheet
54 : Side top sheet
54A : Front side top sheet
54B : Rear side top sheet
56 : Stretching/contracting sheet
60 : Front elastic member
61, 611-619 : First front elastic member
62, 621-624 : Second front elastic member
63, 631, 632 : Third front elastic member
65 : Central elastic member
70 : Rear elastic member
71, 711-717, 71 a-71 c: First rear elastic member
72, 721-724: Second rear elastic member
80: Side joining portion
85: Adhesive portion
CER: Central contraction region
CR: Central region
ER1: First front contraction region
ER2: Second front contraction region
FCR: Front continuous contraction region
LER: Low contraction region
LER1: First low contraction region
LER2: Second low contraction region
RCR: Rear continuous contraction region
RCR1: First rear continuous contraction region
RCR2: Second rear continuous contraction region
RER: Rear contraction region
RLR: Rear low contraction region
SR: Sheet region
LSR: Low contraction sheet region
HSR: High contraction sheet region
S1: Front waistline region
S2: Rear waistline region
S3: crotch region

## Claims

1. An absorbent article (1) having a width direction (W), a front-back direction (L) orthogonal to the width direction (W), a thickness direction (T) orthogonal to the width direction (W) and the front-back direction (L), the article comprising:
a front waistline region (S1);
a rear waistline region (S2);
a crotch region (S3) sandwiched between the front waistline region (S1) and the rear waistline region (S2) in the front-back direction (L); and
a front exterior body (50A) disposed in the front waistline region (S1),
wherein the front exterior body (50A) includes
a stretching/contracting sheet (56) which is a sheet-like elastic member and has stretchability; and
a thread-like or band-like elastic member (60) disposed to overlap with the stretching/contracting sheet (56) in the thickness direction (T) in a stretched state in the width direction (W),
wherein a sheet region (SR) having contractility in the width direction (W) by at least the stretching/contracting sheet (56) is provided,
wherein the sheet region (SR) includes
high contraction sheet regions (HSR) in which the elastic member (60) is disposed so as to overlap with the stretching/contracting sheet (56) in the thickness direction (T), and
a low contraction sheet region (LSR) having lower contractility than that of the high contraction sheet regions (HSR) in the width direction (W), and
wherein the high contraction sheet region (HSR) and the low contraction sheet region (LSR) are arranged in the width direction (W),
wherein the low contraction sheet region (LSR) is disposed to straddle a center of the front exterior body (50A) in the width direction, and
wherein the high contraction sheet regions (HSR) are respectively located at outer side of the low contraction sheet region (LSR) in the width direction.

2. The absorbent article according to claim 1,
wherein the low contraction sheet region (LSR) is disposed between the high contraction sheet regions (HSR) in the width direction (W).

3. The absorbent article according to claim 1 or 2,
wherein the sheet region (SR) includes
a first region (ER1, LER1) in which the high contraction sheet regions (HSR) and the low contraction sheet region (LSR) are arranged in the width direction (W), and
a second region (FCR) in which only the high contraction sheet regions (HSR) is disposed in the width direction (W), and
wherein the second region (FCR) and at least the low contraction sheet region (LSR) of the first region (ER1, LER1) are arranged in the front-back direction (L).

4. The absorbent article according to any one of claims 1 to 3,
wherein both outer side edges of the low contraction sheet region (LSR) in the width direction are located at outer side in the width direction (W) as the low contraction sheet region (LSR) extends outer side in the front-back direction.

5. The absorbent article according to claim 4,
wherein a length in the width direction (W) of an outer end edge of the low contraction sheet region (LSR) in the front-back direction (L) is twice or more a length in the width direction (W) of an inner end edge of the low contraction sheet region (LSR) in the front-back direction (L).

6. The absorbent article according to claims 4 or 5, further comprising:
an absorbent main body (10) which has an absorbent core (20) and is disposed at least in the crotch region (S3),
wherein at least a part of the outer side edge of the low contraction sheet region (LSR) is located at outer side of an outer side edge of the absorbent main body (10) in the width direction (W).

7. The absorbent article according to any one of claims 4 to 6, further comprising:
an absorbent main body (10) which has an absorbent core (20) and is disposed at least in the crotch region (S3),
wherein a part of the high contraction sheet regions (HSR) overlaps with the absorbent main body (10) in the thickness direction (T).

8. The absorbent article according to claim 7,
wherein a part of the high contraction sheet regions (HSR) overlaps with the absorbent core (20) in the thickness direction (T).

9. The absorbent article according to any one of claims 1 to 8,
wherein one or more cuts are formed in the stretching/contracting sheet (56) that configures the low contraction sheet region (LSR).

10. A sheet member having a width direction (W), a front-back direction (L) orthogonal to the width direction (W), a thickness direction (T) orthogonal to the width direction (W) and the front-back direction (L), the sheet member comprising:
a stretching/contracting sheet (56) which is a sheet-like elastic member and has stretchability; and
a thread-like or band-like elastic member (60) extending in the width direction (W) disposed to overlap with the stretching/contracting sheet (56) in the thickness direction (T) in a stretched state in the width direction (W),
wherein a sheet region (SR) having contractility in the width direction (W) by at least the stretching/contracting sheet (56) is provided,
wherein the sheet region (SR) includes
high contraction sheet regions (HSR) in which the elastic member is disposed so as to overlap with the stretching/contracting sheet (56) in the thickness direction (T), and
a low contraction sheet region (LSR) having lower contractility than that of the high contraction sheet regions (HSR) in the width direction (W), and
wherein the high contraction sheet regions (HSR) and the low contraction sheet region (LSR) are arranged in the width direction (W).

## Patentansprüche

1. Absorbierender Artikel (1) aufweisend eine Breitenrichtung (W), eine zur Breitenrichtung (W) orthogonale Vorne-Hinten-Richtung (L) und eine zur Breitenrichtung (W) und zur Vorne-Hinten-Richtung (L) orthogonale Dickenrichtung (T), wobei der Artikel umfasst:
einen vorderen Taillenbereich (S1);
einen hinteren Taillenbereich (S2);
einen Schrittbereich (S5), der in der Vorne-Hinten-Richtung (L) zwischen dem vorderen Taillenbereich (S1) und dem hinteren Taillenbereich (S2) angeordnet ist; und
einen vorderen äußeren Körper (50A), der im vorderen Taillenbereich (S1) angeordnet ist, wobei der vordere äußere Körper (50A) umfasst:
eine Dehn-/Kontraktionslage (56), die ein flächiges elastisches Element ist und eine Dehnbarkeit aufweist; und
ein faden- oder bandförmiges elastisches Element (60), das so angeordnet ist, dass es die Dehn-/Kontraktionslage (56) in der Dickenrichtung (T) überlappt in einem in der Breitenrichtung (W) gedehnten Zustand,
wobei ein Lagenbereich (SR) vorgesehen ist, der zumindest durch die Dehn-/Kontraktionslage (56) eine Kontraktionsfähigkeit in der Breitenrichtung (W) aufweist,
wobei der Lagenbereich (SR) umfasst:
Hochkontraktionslagenbereiche (HSR), in denen das elastische Element (60) so angeordnet ist, dass es die Dehn-/Kontraktionslage (56) in der Dickenrichtung (T) überlappt, und
einen Niedrigkontraktionslagenbereich (LSR), der in der Breitenrichtung (W) eine geringere Kontraktionsfähigkeit als die Hochkontraktionslagenbereiche (HSR) aufweist,
und wobei die Hochkontraktionslagenbereiche (HSR) und der Niedrigkontraktionslagenbereich (LSR) in der Breitenrichtung (W) angeordnet sind,
wobei der Niedrigkontraktionslagenbereich (LSR) so angeordnet ist, dass er ein Zentrum des vorderen äußeren Körpers (50A) in der Breitenrichtung (W) übergreift, und
wobei die Hochkontraktionslagenbereiche (HSR) jeweils an den äußeren Seiten des Niedrigkontraktionslagenbereichs (LSR) in der Breitenrichtung (W) angeordnet sind.

2. Absorbierender Artikel nach Anspruch 1, **dadurch gekennzeichnet, dass** der Niedrigkontraktionslagenbereich (LSR) in der Breitenrichtung (W) zwischen den Hochkontraktionslagenbereichen (HSR) angeordnet ist.

3. Absorbierender Artikel nach Anspruch 1 oder 2, wobei der Lagenbereich (SR) umfasst:
einen ersten Bereich (ER1, LER1), in dem die Hochkontraktionslagenbereiche (HSR) und der Niedrigkontraktionslagenbereich (LSR) in der Breitenrichtung (W) angeordnet sind, und
einen zweiten Bereich (FCR), in dem in der Breitenrichtung (W) ausschließlich die Hochkontraktionslagenbereiche (HSR) angeordnet sind,
wobei der zweite Bereich (FCR) und zumindest der Niedrigkontraktionslagenbereich (LSR) des ersten Bereichs (ER1, LER1) in der Vorne-Hinten-Richtung (L) angeordnet sind.

4. Absorbierender Artikel nach einem der Ansprüche 1 bis 3, **dadurch gekennzeichnet, dass** beide äußeren Seitenkanten des Niedrigkontraktionslagenbereichs (LSR) in der Breitenrichtung außenseitig in der Breitenrichtung (W) angeordnet sind, während sich der Niedrigkontraktionslagenbereich (LSR) in der Vorne-Hinten-Richtung (L) nach außen erstreckt.

5. Absorbierender Artikel nach Anspruch 4, **dadurch gekennzeichnet, dass** eine Länge in der Breitenrichtung (W) einer äußeren Endkante des Niedrigkontraktionslagenbereichs (LSR) in der Vorne-Hinten-Richtung (L) das Doppelte oder mehr einer Länge in der Breitenrichtung (W) einer inneren Endkante des Niedrigkontraktionslagenbereichs (LSR) in der Vorne-Hinten-Richtung (L) beträgt.

6. Absorbierender Artikel nach Anspruch 4 oder 5, weiter umfassend:
einen absorbierenden Hauptkörper (10), der einen Absorptionskern (20) aufweist und zumindest im Schrittbereich (SS) angeordnet ist,
wobei zumindest ein Teil der äußeren Seitenkante des Niedrigkontraktionslagenbereichs (LSR) in der Breitenrichtung (W) an der äußeren Seite einer äußeren Seitenkante des absorbierenden Hauptkörpers (10) angeordnet ist.

7. Absorbierender Artikel nach einem der Ansprüche 4 bis 6, weiter umfassend:
einen absorbierenden Hauptkörper (10), der einen Absorptionskern (20) aufweist und zumindest im Schrittbereich (SS) angeordnet ist,
wobei ein Teil der Hochkontraktionslagenbereiche (HSR) den absorbierenden Hauptkörper (10) in der Dickenrichtung (T) überlappt.

8. Absorbierender Artikel nach Anspruch 7, **dadurch gekennzeichnet, dass** ein Teil der Hochkontraktionslagenbereiche (HSR) den Absorptionskern (20) in der Dickenrichtung (T) überlappt.

9. Absorbierender Artikel nach einem der Ansprüche 1 bis 8, **dadurch gekennzeichnet, dass** ein oder mehrere Schnitte in der Dehn-/Kontraktionslage (56) ausgebildet sind, welche den Niedrigkontraktionslagenbereich (LSR) bildet.

10. Lagenbauteil mit einer Breitenrichtung (W), einer zur Breitenrichtung (W) orthogonalen Vorne-Hinten-Richtung (L) sowie einer zur Breitenrichtung (W) und zur Vorne-Hinten-Richtung (L) orthogonalen Dickenrichtung (T), wobei das Lagenbauteil umfasst:
eine Dehn-/Kontraktionslage (56), die ein flächiges elastisches Element ist und eine Dehnbarkeit aufweist; und
ein faden- oder bandförmiges elastisches Element (60), das sich in der Breitenrichtung (W) erstreckt und in einem gedehnten Zustand in der Breitenrichtung (W) so angeordnet ist, dass es die Dehn-/Kontraktionslage (56) in der Dickenrichtung (T) überlappt,
wobei ein Lagenbereich (SR) vorgesehen ist, der zumindest durch die Dehn-/Kontraktionslage (56) eine Kontraktionsfähigkeit in der Breitenrichtung (W) aufweist,
wobei der Lagenbereich (SR) umfasst:
Hochkontraktionslagenbereiche (HSR), in denen das elastische Element so angeordnet ist, dass es die Dehn-/Kontraktionslage (56) in der Dickenrichtung (T) überlappt, und
einen Niedrigkontraktionslagenbereich (LSR), der in der Breitenrichtung (W) eine geringere Kontraktionsfähigkeit als die Hochkontraktionslagenbereiche (HSR) aufweist,
wobei die Hochkontraktionslagenbereiche (HSR) und der Niedrigkontraktionslagenbereich (LSR) in der Breitenrichtung (W) angeordnet sind.

## Revendications

1. Article absorbant (1) ayant une direction de la largeur (W), une direction avant-arrière (L) orthogonale à la direction de la largeur (W), une direction de l'épaisseur (T) orthogonale à la direction de la largeur (W) et à la direction avant-arrière (L), l'article comprenant :
une région de ceinture avant (S1) ;
une région de ceinture arrière (S2) ;
une région d'entrejambe (S3) prise en sandwich entre la région de ceinture avant (S1) et la région de ceinture arrière (S2) dans la direction avant-arrière (L) ; et
un corps extérieur avant (50A) disposé dans la région de ceinture avant (S1),
dans lequel le corps extérieur avant (50A) inclut
une feuille d'étirage/contraction (56) qui est un élément élastique de type feuille et présente une extensibilité ; et
un élément élastique (60) de type fil ou de type bande disposé de façon à chevaucher la feuille d'étirage/contraction (56) dans la direction de l'épaisseur (T) dans un état étiré dans la direction de la largeur (W),
dans lequel une région de feuille (SR) ayant une contractilité dans la direction de la largeur (W) par au moins la feuille d'étirage/contraction (56) est prévue,
dans lequel la région de feuille (SR) inclut
des régions de feuille à haute contraction (HSR) dans lesquelles l'élément élastique (60) est disposé de manière à chevaucher la feuille d'étirage/contraction (56) dans la direction de l'épaisseur (T), et
une région de feuille à faible contraction (LSR) ayant une contractilité inférieure à celle des régions de feuille à haute contraction (HSR) dans la direction de la largeur (W), et
dans lequel la région de feuille à haute contraction (HSR) et la région de feuille à faible contraction (LSR) sont agencées dans la direction de la largeur (W),
dans lequel la région de feuille à faible contraction (LSR) est disposée de façon à enjamber un centre du corps extérieur avant (50A) dans la direction de la largeur, et
dans lequel les régions de feuille à haute contraction (HSR) sont respectivement situées au niveau d'un côté extérieur de la région de feuille à faible contraction (LSR) dans la direction de la largeur.

2. Article absorbant selon la revendication 1,
dans lequel la région de feuille à faible contraction (LSR) est disposée entre les régions de feuille à haute contraction (HSR) dans la direction de la largeur (W).

3. Article absorbant selon la revendication 1 ou 2,
dans lequel la région de feuille (SR) inclut
une première région (ER1, LER1) dans laquelle les régions de feuille à haute contraction (HSR) et la région de feuille à faible contraction (LSR) sont agencées dans la direction de la largeur (W), et
une seconde région (FCR) dans laquelle seule les régions de feuille à haute contraction (HSR) est disposée dans la direction de la largeur (W), et
dans lequel la seconde région (FCR) et au moins la région de feuille à faible contraction (LSR) de la première région (ER1, LER1) sont agencées dans la direction avant-arrière (L).

4. Article absorbant selon l'une quelconque des revendications 1 à 3,
dans lequel les deux bords latéraux extérieurs de la région de feuille à faible contraction (LSR) dans la direction de la largeur sont situés au niveau d'un côté extérieur dans la direction de la largeur (W) à mesure que la région de feuille à faible contraction (LSR) s'étend vers le côté extérieur dans la direction avant-arrière.

5. Article absorbant selon la revendication 4,
dans lequel une longueur dans la direction de la largeur (W) d'un bord d'extrémité extérieur de la région de feuille à faible contraction (LSR) dans la direction avant-arrière (L) est égale ou supérieure à deux fois une longueur dans la direction de la largeur (W) d'un bord d'extrémité intérieur de la région de feuille à faible contraction (LSR) dans la direction avant-arrière (L).

6. Article absorbant selon les revendications 4 ou 5, comprenant en outre :
un corps principal absorbant (10) qui possède une âme absorbante (20) et est disposé au moins dans la région d'entrejambe (S3),
dans lequel au moins une partie du bord latéral extérieur de la région de feuille à faible contraction (LSR) est située au niveau d'un côté extérieur d'un bord latéral extérieur du corps principal absorbant (10) dans la direction de la largeur (W).

7. Article absorbant selon l'une quelconque des revendications 4 à 6, comprenant en outre :
un corps principal absorbant (10) qui possède une âme absorbante (20) et est disposé au moins dans la région d'entrejambe (S3),
dans lequel une partie des régions de feuille à haute contraction (HSR) chevauche le corps principal absorbant (10) dans la direction de l'épaisseur (T).

8. Article absorbant selon la revendication 7,
dans lequel une partie des régions de feuille à haute contraction (HSR) chevauche l'âme absorbante (20) dans la direction de l'épaisseur (T).

9. Article absorbant selon l'une quelconque des revendications 1 à 8,
dans lequel une ou plusieurs découpes sont formées dans la feuille d'étirage/contraction (56) qui configure la région de feuille à faible contraction (LSR).

10. Élément de feuille ayant une direction de la largeur (W), une direction avant-arrière (L) orthogonale à la direction de la largeur (W), une direction de l'épaisseur (T) orthogonale à la direction de la largeur (W) et à la direction avant-arrière (L), l'élément de feuille comprenant :
une feuille d'étirage/contraction (56) qui est un élément élastique de type feuille et présente une extensibilité ; et
un élément élastique (60) de type fil ou de type bande s'étendant dans la direction de la largeur (W) disposé de façon à chevaucher la feuille d'étirage/contraction (56) dans la direction de l'épaisseur (T) dans un état étiré dans la direction de la largeur (W),
dans lequel une région de feuille (SR) ayant une contractilité dans la direction de la largeur (W) par au moins la feuille d'étirage/contraction (56) est prévue,
dans lequel la région de feuille (SR) inclut
des régions de feuille à haute contraction (HSR) dans lesquelles l'élément élastique est disposé de manière à chevaucher la feuille d'étirage/contraction (56) dans la direction de l'épaisseur (T), et
une région de feuille à faible contraction (LSR) ayant une contractilité inférieure à celle des régions de feuille à haute contraction (HSR) dans la direction de la largeur (W), et dans lequel les régions de feuille à haute contraction (HSR) et la région de feuille à faible contraction (LSR) sont agencées dans la direction de la largeur (W).
